(19) Europäisches Patentamt
European Patent Office
Office européen des brevets

(11) **EP 4 154 818 B1**

(12) **EUROPÄISCHE PATENTSCHRIFT**

(45) Veröffentlichungstag und Bekanntmachung des Hinweises auf die Patenterteilung:
**07.01.2026  Patentblatt 2026/02**

(21) Anmeldenummer: **22190551.6**

(22) Anmeldetag: **16.08.2022**

(51) Internationale Patentklassifikation (IPC):
**A61B 6/00** *(2024.01)*        **A61B 6/50** *(2024.01)*
**A61B 6/02** *(2006.01)*

(52) Gemeinsame Patentklassifikation (CPC):
**A61B 6/5217; A61B 6/505;** A61B 6/025;
A61B 6/482

(54) **COMPUTERIMPLEMENTIERTES VERFAHREN ZUR ERMITTLUNG EINER DIE SKELETTREIFE EINES PATIENTEN BESCHREIBENDEN KNOCHENINFORMATION, RÖNTGENEINRICHTUNG, ERMITTLUNGSEINRICHTUNG, COMPUTERPROGRAMM UND ELEKTRONISCH LESBARER DATENTRÄGER**

COMPUTER-IMPLEMENTED METHOD FOR DETERMINING BONE INFORMATION DESCRIBING THE SKELETAL MATURITY OF A PATIENT, X-RAY DEVICE, DETERMINATION DEVICE, COMPUTER PROGRAM AND ELECTRONICALLY READABLE DATA CARRIER

PROCÉDÉ MIS EN ŒUVRE PAR ORDINATEUR DESTINÉ À LA DÉTERMINATION DES INFORMATIONS OSSEUSES DÉCRIVANT LA MATURITÉ DU SQUELETTE D'UN PATIENT, DISPOSITIF À RAYONS X, DISPOSITIF DE DÉTERMINATION, PROGRAMME INFORMATIQUE ET SUPPORT DE DONNÉES LISIBLE DE MANIÈRE ÉLECTRONIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **24.09.2021  DE 102021210675**

(43) Veröffentlichungstag der Anmeldung:
**29.03.2023  Patentblatt 2023/13**

(73) Patentinhaber: **Siemens Healthineers AG
91301 Forchheim (DE)**

(72) Erfinder:
• **Fieselmann, Andreas
91052 Erlangen (DE)**
• **Hörnig, Mathias
91096 Möhrendorf (DE)**

(74) Vertreter: **Siemens Healthineers
Patent Attorneys
Postfach 22 16 34
80506 München (DE)**

(56) Entgegenhaltungen:
**WO-A1-2018/057714**

• **VAN HOUWELINGEN ILVA: "Automated Bone Age Assessment based on DXA scans for a variety of ethnicities using Deep Transfer Learning MSc Thesis Biomedical Engineering -Medical Physics -BM51035", DEEP TRANSFER LEARNING, 24 June 2021 (2021-06-24), XP055957847**
• **MAGNIDE EDDIE ET AL: "Automatic bone maturity grading from EOS radiographs in Adolescent Idiopathic Scoliosis", COMPUTERS IN BIOLOGY AND MEDICINE, NEW YORK, NY, US, vol. 136, 23 July 2021 (2021-07-23), XP086773803, ISSN: 0010-4825, [retrieved on 20210723], DOI: 10.1016/ J.COMPBIOMED.2021.104681**

**Beschreibung**

Computerimplementiertes Verfahren zur Ermittlung einer die Skelettreife eines Patienten beschreibenden Knocheninformation, Röntgeneinrichtung, Ermittlungseinrichtung, Computerprogramm und elektronisch lesbarer Datenträger

Die Erfindung betrifft ein computerimplementiertes Verfahren zur Ermittlung einer die Skelettreife eines Patienten beschreibenden Knocheninformation, eine Röntgeneinrichtung, eine Ermittlungseinrichtung, ein Computerprogramm und einen elektronisch lesbaren Datenträger.

Skoliose ist eine Krankheit, bei der die Wirbelsäule nicht der natürlichen, geradlinigen Form in der Frontalebene folgt. Anders gesagt handelt es sich um eine Seitabweichung der Wirbelsäule von der Längsachse, bei der eine Verdrehung der Wirbel und/oder Torsion der Wirbelkörper auftreten kann. Die idiopathische Adoleszentenskoliose (adolescent idiopathic scoliosis - AIS) ist die am häufigsten vorkommende Form der Skoliose. Sie betrifft etwa 1 - 4 % der Heranwachsenden. Bei der AIS, aber auch bei anderen Knochenkrankheiten, müssen Ärzte patientenspezifisch entscheiden, welche Behandlung am geeignetsten ist. Dabei ist es zum einen kritisch, zu wenige Behandlungsmaßnahmen, beispielsweise verspätete chirurgische Eingriffe, zu vermeiden, zum anderen aber auch, Überbehandlungen, beispielsweise durch einen chirurgischen Eingriff, wenn er nicht benötigt wird, zu verhindern. Bei der AIS schätzen Experten das Risiko einer Verschlimmerung der Formabweichung ab, insbesondere das Risiko, dass ohne Behandlung eine noch unkritische skoliotische Verformung zu einer kritischen skoliotischen Verformung fortschreitet, vergleiche beispielsweise den Artikel von Sabrina Donzelli et al. "Predicting scoliosis progression: a challenge for researchers and clinicians", EClinicalMedicine 18:100244, 2020.

Um das Risiko eines Fortschreitens einer Skoliose abzuschätzen, können Bildgebung, aber auch nicht durch Bildgebung ermittelbare Biomarker verwendet werden. Als durch Bildgebung gewinnbare Biomarker können häufig der sogenannte Cobb-Winkel und die Skelettreife verwendet werden. Der Cobb-Winkel beschreibt die Stärke der Verformung anhand des Winkels von Geraden durch die oberhalb und unterhalb des Apex-Wirbels am stärksten verdrehten Wirbel. Zur Beschreibung der Skelettreife (englisch "skeletal maturity" oder "bone maturity") existieren verschiedene Ansätze. Unter der Skelettreife wird der Grad der Reifung der Knochen eines Kindes verstanden. Zur Bewertung der Skelettreife kann beispielsweise ein Röntgenbild der linken Hand, der Finger und des Handgelenks aufgenommen werden. Dann können die sichtbaren Knochen mit einem Standartatlas, beispielsweise "Greulich und Pyle" verglichen werden. Ein anderer Ansatz bezüglich der linken Hand betrifft die Sanders-Reifeskala (sanders maturity scale, SMS). Weitere Hinweise können sich auch durch Röntgenbildgebung der Ulna (Elle) oder des proximalen Humerus (Oberarmknochen) ergeben. Ein gerade bei der Betrachtung der Skoliose häufig verwendeter Ansatz ist die Ermittlung des Risser-Zeichens, die die Verknöcherung (Ossifizierung) am Beckenkamm (konkret Darmbeinkamm/Iliumkamm) beschreibt, nachdem bei der Bildgebung der Wirbelsäure häufig auch das Becken aufgenommen wird. Diesbezüglich sei auch auf den Artikel von Maximilian Lenz et al., "Scoliosis and Prognosis - a systematic review regarding patient-specific and radiological predictive factors for curve progression", European Spine Journal, doi:10.1007/s00586-021-06817-0, 26.03.2021, verwiesen.

Die Bestimmung des Risser-Zeichens wird häufig genutzt, um die Skelettreife für die Einschätzung des Wirbelsäulenwachstumspotenzials und das Verschlimmerungsrisiko bei Skoliose einzuschätzen. Dabei sind meist keine zusätzlichen Röntgenbilder erforderlich, nachdem der Beckenbereich üblicherweise Teil einer Standard-Wirbelsäulen-Röntgenuntersuchung zur Skolioseauswertung ist. Basierend auf der Verknöcherung des Beckenkamms (auch Darmbeinkamm oder englisch "iliac crest") wird ein Wert von 0 bis 5 vergeben. Studien, beispielsweise ein Artikel von Jae Hyuk Yang et al. "Evaluation of accuracy of plain radiography in determining the Risser stage and identification of common sources of errors", Journal of Orthopaedic Surgery and Research 2014, 9:101, haben gezeigt, dass die Abschätzung des Risser-Zeichens durch einfache Radiographie nicht akkurat ist. Variationen in der Verknöcherung der Ilium-Apophyse und Fehlinterpretation der Apophysenfusion sind die hauptsächlichen Quellen von Fehlern.

Auch bei der Betrachtung anderer Zeichen und Skelettreifemaße können derartige Unsicherheiten auftreten. Verschiedene Ansätze sind, wie bereits erwähnt, in dem zitierten Artikel von Maximilian Lenz et al. gegeben.

Skelettreifemaße, insbesondere das Risser-Zeichen, werden heute meist visuell basierend auf einfachen Radiographien bestimmt. Um eine verbesserte Bestimmung des Risser-Zeichens zu ermöglichen, wurde in einem Artikel von Houda Kaddioui et al. "Convolutional Neural Networks for Automatic Risser Stage Assessment", Radiology: Artificial Intelligence, 2(3), 2020, vorgeschlagen, übliche Radiographien durch künstliche Intelligenz auszuwerten, was jedoch nicht immer zu verlässlichen und genauen Ergebnissen führt. In einem Artikel von Martin Thaler et al. "Radiographic versus ultrasound evaluation of the Risser Grade in adolescent idiopathic scoliosis: a prospective study of 46 patients" European Spine Journal, 17(9): 1251-1255, wurde vorgeschlagen, statt Röntgenbildgebung Ultraschallbildgebung einzusetzen, allerdings wird hier zusätzliche Untersuchungszeit benötigt.

Aus der Veröffentlichung VAN HOUWELINGEN, Ilva. Automated Bone Age Assessment based on DXA scans for a variety of ethnicities using Deep Transfer Learning. 2021. ist ein automatisches Verfahren zu Bestimmung des Knochenalters basierend auf DXA-Daten unter Verwendung einer trainierten Auswertungsfunktion bekannt.

Der Erfindung liegt die Aufgabe zugrunde, eine möglichst robuste, akkurate und wenig aufwendige Methode zum Ermitteln einer Knocheninformation, insbesondere eines Skelettreifemaßes, anzugeben.

Diese Aufgabe wird erfindungsgemäß gelöst durch ein computerimplementiertes Verfahren, eine Röntgeneinrichtung, eine Ermittlungseinrichtung, ein Computerprogramm und einen elektronisch lesbaren Datenträger gemäß den unabhängigen Ansprüchen. Vorteilhafte Ausgestaltungen ergeben sich aus den Unteransprüchen.

Ein erfindungsgemäßes computerimplementiertes Verfahren zur Ermittlung einer die Skelettreife eines Patienten beschreibenden Knocheninformation umfasst die Schritte:

- Bereitstellung von Eingangsdaten, umfassend spektral aufgelöste Röntgendaten eines zur Beurteilung der Skelettreife heranzuziehenden interessierenden Bereichs des Patienten mit wenigstens zwei auf unterschiedliche Röntgenspektren bezogenen Röntgenbilddatensätzen,
- Anwenden einer trainierten Auswertungsfunktion auf die Eingangsdaten zum Erhalt der Knocheninformation als Ausgangsdaten, und
- Ausgabe der Knocheninformation.

Spektrale Röntgenbildgebung (oft auch Mehrenergie-Röntgenbildgebung) ist im Stand der Technik grundsätzlich bereits bekannt. Hierbei wird nicht nur ein einziger Röntgenbilddatensatz erhalten, sondern es werden mehrere, unterschiedlichen Röntgenspektren, insbesondere unterschiedlichen mitteleren Röntgenenergien, zugeordnete Röntgenbilddatensätze erzeugt. Dies kann grundsätzlich dadurch geschehen, dass ein Röntgenstrahler zeitlich sukzessive zur Ausstrahlung unterschiedlicher Strahlungsspektren, beispielsweise unter Verwendung unterschiedlicher Röhrenspannungen, angesteuert wird und zeitlich nacheinander die Röntgenbilddatensätze aufgenommen werden. Im Rahmen der vorliegenden Erfindung ist es jedoch, worauf noch näher eingegangen werden wird, bevorzugt, detektorbasierte spektrale Röntgenbildgebung zu nutzen, da der Röntgendetektor spektral selektiv ausgestaltet ist, mithin unterschiedliche Röntgenenergien unterscheiden kann und unterschiedlichen Röntgenspektren zugeordnete Röntenbilddatensätze in einem einzigen, gemeinsamen Aufnahmevorgang erzeugen kann, sodass Bewegungsartefakte vermieden werden und eine reduzierte Aufnahmezeit gegeben ist. Dabei wird im Rahmen der vorliegenden Erfindung bevorzugt Dualenergie-Röntgenbildgebung verwendet, so dass mithin zwei jeweils Röntgendaten für ein unterschiedliches Röntgenspektrum zeigende Röntgenbilddatensätze aufgenommen werden, insbesondere ein Hochenergie-Röntgenbilddatensatz und ein Niedrigenergie-Röntgenbilddatensatz.

Im Unterschied zum Stand der Technik wird also vorgeschlagen, die Knocheninformation automatisch mit spektral aufgelösten Röntgendaten zu ermitteln, wobei künstliche Intelligenz in Form der trainierten Auswertungsfunktion eingesetzt wird. Dem liegt die Idee zugrunde, dass hierdurch nicht nur die Knochenmorphologie hervorragend beschrieben wird, die sich beispielsweise aus dem Hochenergie-Röntgenbilddatensatz ergeben kann, sondern auch Informationen zur Knochendichte bzw. zum Knochenmineralgehalt (bone mineral content - BMC) enthalten sind. Dem liegt wiederum die Erkenntnis zugrunde, dass die Skelettreife durch den Knochenwachstumsprozess beeinflusst wird, welcher wiederum auf dem lokalen Knochenmineralgehalt beruht. Der Knochenmineralgehalt ist die Mineralkomponente des Knochens in Form von Hydroxylapatit, dessen Bewertung durch Mehrenergiebildgebung, insbesondere bereits Dualenergie-Röntgenbildgebung, möglich ist.

Daher sieht eine besonders bevorzugte Weiterbildung der vorliegenden Erfindung vor, dass als Eingangsdaten auch ein aus den Röntgenbilddatensätzen ermittelter, Knochenmineralien hervorhebender Kombinationsbilddatensatz ermittelt wird, insbesondere durch gewichtete Linearkombination der Röntgenbilddatensätze. Beispielsweise im Hinblick auf die Osteoporose-Bewertung wurde im Stand der Technik bereits vorgeschlagen, ein relatives Maß für den Knochenmineralgehalt durch Kombination von unterschiedlichen Röntgenspektren zugeordneten Röntgenbilddatensätzen zu ermitteln. In diesem Zusammenhang wurde insbesondere die sogenannte Dualenergie-Röntgen-Absorptiometrie (dual energy X-ray absorptiometry - DXA) vorgeschlagen. In diesem Zusammenhang sei insbesondere auf die Veröffentlichung "Dual Energy X-ray Absorptiometry for Bone Mineral Density and Body Composition Assessment", 2010, der IAEA verwiesen, abrufbar unter https://www-pub.iaea.org/MTCD/Publications/PDF/Pub1479_web.pdf.

Es kann also gesagt werden, dass die unterschiedlichen Mehrenergie-Röntgenbilddatensätze zueinander komplementäre Informationen enthalten, so dass wenigstens zwei relevante Eigenschaften für die Skelettreife aus demselben Scan des Patienten hergeleitet werden können. Der Einsatz künstlicher Intelligenz in Form der trainierten Auswertungsfunktion erlaubt es in diesem Zusammenhang, tatsächlich alle relevanten Merkmale zur Bestimmung der Knocheninformation heranzuziehen, mithin auch Aspekte zu berücksichtigen, die für den Betrachter visuell aus den Röntgenbilddatensätzen nicht ersichtlich wären. Auf diese Weise wird eine hochqualitative, robuste, akkurate und verlässliche Bestimmung der die Skelettreife beschreibenden Knocheninformation ermöglicht.

Wie bereits erwähnt, wird erfindungsgemäß die Verwendung von detektorbasierter spektraler Röntgenbildgebung bevorzugt. Eine bevorzugte Ausgestaltung der vorliegenden Erfindung sieht mithin vor, dass die Röntgendaten mittels eines spektral selektiven Röntgendetektors, insbesondere eines mehrere, unterschiedliche Energiebereiche vermessende Schichten aufweisenden Mehrschichtdetektors, aufgenommen werden. Ein derartiger Mehrschichtdetektor kann beispielsweise ein 2-Schicht-Detektor (dual layer detector) sein. Ein 2-Schicht-Detektor ist ausgestaltet, das Spektrum der einfallenden Röntgenstrahlung in einen niederenergetischen und einen hochenergetischen Anteil zu zerlegen. Dazu ist der 2-Schicht-Detektor aus zwei Schichten aufgebaut, wobei eine dem Röntgenstrahler zugewandte Detektorschicht Röntgenphotonen der einfallenden Röntgenstrahlung, die niedrige Energie aufweisen, misst. Die gemessenen Signale werden einem ersten (Niedrigenergie-)Röntgenbilddatensatz zugeordnet. Die erste Detektorschicht ist transparent für hochenergetische Röntgenstrahlung, sodass Röntgenphotonen mit höherer Quantenenergie in der darunter bzw. dahinter, also von dem Röntgenstrahler abgewandten, zweiten Detektorschicht gemessen werden können. Diese gemessenen Signale werden entsprechend einem zweiten (Hochenergie-)Röntgenbilddatensatz zugeordnet. Beispielsweise können beide Detektorschichten einen Szintillator umfassen, sodass es sich bei dem 2-Schicht-Detektor um einen indirekt konvertierenden Detektor handelt. Insbesondere können Kristalle wie Cäsiumjodit, Kadmiumwolframat oder keramische Stoffe, beispielsweise Gadoliniumoxysulfid, als Detektormaterialien zum Einsatz kommen.

Eine andere Art von spektral selektivem Röntgendetektor, der im Rahmen der vorliegenden Erfindung eingesetzt werden kann, ist ein photonenzählender Röntgendetektor, der beispielsweise keinen Szintillator verwenden kann, sondern einen Halbleiter verwenden kann, der Röntgenphotonen direkt detektiert. Daher werden solche Detektoren auch direkt konvertierende Detektoren genannt. Auf die Detektorfläche einfallende Röntgenphotonen können je nach ihrer Quantenenergie bestimmten Energiebändern bzw. Energie-Bins zugeordnet werden.

Zweckmäßigerweise können jeweils wenigstens zwei zweidimensionale und/oder wenigstens zwei dreidimensionale Röntgenbilddatensätze für die unterschiedlichen Spektren bereitgestellt werden. Dreidimensionale Röntgenbilddatensätze, die im Rahmen der vorliegenden Erfindung bevorzugt mittels 3D-Tomosynthese aufgenommen werden, haben den Vorteil, dass die Genauigkeit der Bestimmung der Knocheninformation weiter verbessert werden kann, da überlappende Strukturen im Vergleich zu zweidimensionalen Röntgenbilddatensätzen reduziert werden können.

Dabei kann es durchaus vorgesehen sein, sowohl zweidimensionale als auch dreidimensionale Röntgenbilddatensätze als Eingangsdaten für die trainierte Auswertungsfunktion zu verwenden. Die zweidimensionalen Röntgenbilddatensätze werden bevorzugt mittels Schlitzscannen (englisch slot scanning) aufgenommen. Dabei werden wiederholt schmale Aufnahmen benachbarter Teilbereiche durch Verwendung eines schlitzartigen Kollimators durchgeführt, welcher senkrecht zur Längsrichtung des Schlitzes über die Detektorfläche des Röntgendetektors bewegt werden kann. Es wird also immer nur ein schmaler Abschnitt aufgenommen. Dies hat den Vorteil, dass weniger Streustrahlung auf den Röntgendetektor auftrifft, mithin Streustrahlungseffekte zur Erhöhung der Bildqualität und somit der Qualität der Knocheninformation reduziert werden, zudem aber auch eine Aufnahmegeometrie gegeben ist, in der die Röntgenstrahlung bei zweckmäßig mitbewegten Röntgenstrahler eher senkrecht auf den gerade aufzunehmenden Bereich trifft. Hinsichtlich dreidimensionaler Röntgenbilddatensätze wird vorgeschlagen, diese durch Tomosynthese aufzunehmen, welche eine bereits seit langem bekannte Technik darstellt, bei der eine reduzierte Anzahl von Projektionsrichtungen verwendet wird, was die Röntgenbelastung für den Patienten vorteilhafterweise möglichst gering hält.

In der Erfindung ist vorgesehen, dass die Röntgendaten wenigstens teilweise als Teil oder im Rahmen eines einen größeren Aufnahmebereich als den interessierenden Bereich betreffenden Untersuchungsvorgangs aufgenommen werden. Dabei kann der Aufnahmebereich insbesondere, beispielsweise zur Untersuchung hinsichtlich einer Skoliose, die Wirbelsäule insgesamt betreffen, der interessierende Bereich den Beckenbereich. Dies hat den Vorteil, dass für die Röntgendaten kein zusätzlicher weiterer Untersuchungsvorgang benötigt wird. Insbesondere sogar für einen anderen Zweck, beispielsweise hinsichtlich des Vorliegens einer Skoliose, aufgenommene Röntgenbilder können auch als Röntgendaten weiterverwendet werden. Dies bedeutet also insbesondere, dass in dem Untersuchungsvorgang bezüglich des größeren Aufnahmebereichs aufgenommene Röntgenbilder auch als Röntgendaten verwendet werden können. Durch die mehrfache Verwendung dieser Röntgendaten kann mithin ebenso Dosis für den Patienten eingespart werden. Zudem existieren Vorteile bezüglich der insgesamten Untersuchungszeit. Betrifft beispielsweise der Aufnahmebereich die Wirbelsäule, können die Röntgenbilder der Gesamtuntersuchung genutzt werden, um das Vorliegen und die Stärke einer Skoliose zu beurteilen, beispielsweise durch Bestimmung des Cobb-Winkels. Gleichzeitig kann aber der in diesem Zusammenhang üblicherweise auch aufgenommene Beckenbereich genutzt werden, um die Knocheninformation bezüglich der Skelettreife automatisch mittels der trainierten Auswertungsfunktion herzuleiten und beispielsweise für eine Risikoabschätzung hinsichtlich des Fortschreitens der Skoliose zu berücksichtigen. Auch wenn sich das hauptsächlich diskutierte Ausführungsbeispiel auf die Wirbelsäule und den Beckenbereich, insbesondere hinsichtlich des Risser-Zeichens als Skelettreifemaß, beziehen mag, lässt sich das hier beschriebene Konzept jedoch allgemeiner einsetzen. Beispielsweise können die Mehrenergie-Informationen hinsichtlich der Knochenmorphologie und der Knochendichte/des Knochenmineralgehalts auch für interessierende Bereiche bzw. Aufnahmebereiche im Bereich des Humerus, des Radius, der Ulna und/oder der Hand genutzt werden.

Konkret ist vorgesehen, dass wenigstens ein zweidimensional aufgenommenes Röntgenbild des Untersuchungsvorgangs und/oder eine empfangene Nutzerinformation zur Lokalisierung des interessierten Bereichs ausgewertet werden, wobei das Auswertungsergebnis zur nachfolgenden oder unterbrechenden Aufnahme dreidimensionaler, nur auf den interessierenden Bereich bezogener dreidimensionaler Röntgendaten und/oder zur Auswahl von als Eingangsdaten zu verwendenden zweidimensionalen Röntgendaten der Röntgenbilder verwendet werden. Dabei wird erfindungsgemäß bevorzugt, während oder nach dem Untersuchungsvorgang in den Röntgenbildern festzustellen, wann der interessierende Bereich erreicht ist bzw. wo sich dieser in Bezug auf den Aufnahmebereich befindet. Denkbar ist es jedoch auch, die Lokalisierung wenigstens teilweise auf der Grundlage einer empfangenen Nutzerinformation durchzuführen, wozu beispielsweise das wenigstens eine Röntgenbild dem Nutzer zur Anzeige gebracht werden kann, wo dieser die Lage des interessierenden Bereichs markieren oder eine bildbasiert festgestellte Markierung bestätigen kann. Die so erhaltene Lokalisierungsinformation kann beispielsweise genutzt werden, um aus den Röntgenbildern als zweidimensionale Röntgendaten zu verwendende Anteile auszuwählen und an die trainierte Auswertungsfunktion weiterzugeben. Jedoch ist es auch denkbar, die Aufnahme wenigstens eines zweidimensionalen Röntgenbildes zu unterbrechen und, insbesondere durch Tomosynthese, nach Erreichen des interessierenden Bereichs dreidimensionale Röntgendaten des interessierenden Bereichs aufzunehmen, die als Eingangsdaten verwendet werden können. Selbstverständlich ist es auch möglich, die Aufnahme der dreidimensionalen Röntgendaten erst nach Abschluss des Untersuchungsvorgangs bezüglich des Aufnahmebereichs vorzunehmen, dann ebenso bevorzugt als Tomosynthese.

Im Zusammenhang der Unterbrechung sieht eine besonders vorteilhafte Ausgestaltung der vorliegenden Erfindung vor, dass aus den dreidimensionalen Röntgendaten ein zweidimensionales Synthetikbild des interessierenden Bereichs ermittelt wird, welches mit dem wenigstens einen Röntgenbild fusioniert wird. Für diesen Bereich müssen dann keine zweidimensionalen Röntgenbilder aufgenommen werden. In anderen Worten kann ein synthetisches, radiographisches Röntgenbild - das Synthetikbild - aus den dreidimensionalen Röntgendaten hergeleitet werden und mit dem wenigstens einen aufgenommenen zweidimensionalen Röntgenbild, welches bevorzugt durch einen Schlitzscan aufgenommen wird, fusioniert werden. Auf diese Weise kann weiterhin Röntgendosis eingespart werden, nachdem für den interessierenden Bereich ein Synthetikbild berechnet und verwendet werden kann.

Im Folgenden werden drei konkrete Varianten zur Aufnahme der Röntgendaten beispielhaft näher dargestellt. In einer ersten Variante kann, insbesondere unter Verwendung des spektral selektiven Röntgendetektors, eine Aufnahme des wenigstens einen spektralen Röntgenbildes des Aufnahmebereichs erfolgen. Dies geschieht bevorzugt durch Schlitzscannen. Beispielsweise können für einen die Wirbelsäule betreffenden Aufnahmebereich durch drei aufeinander folgende Schlitzscans drei zweidimensionale Röntgenbilder aufgenommen und zu einem Gesamtbild fusioniert werden. Bevorzugt automatisch, aber auch manuell oder aufgrund einer vorherigen Einstellung kann die Lage des interessierenden Bereichs, beispielsweise des Beckenbereichs, ermittelt werden und aus dem Gesamtbild können die zweidimensionalen Röntgendaten, als zwei zweidimensionale Röntgenbilddatensätze, ausgewählt werden, die den interessierenden Bereich zeigen und als Eingangsdaten für die trainierte Auswertungsfunktion verwendet werden. Bevorzugt können hierbei drei Röntgenbilddatensätze verwendet werden, nämlich ein Hochenergie-Röntgenbilddatensatz, ein Niedrigenergie-Röntgenbilddatensatz und ein Kombinationsbilddatensatz, welcher sich insbesondere auf den Knochenmineralgehalt bezieht.

In einer zweiten Variante wird wiederum zunächst ein zweidimensionaler Scan des gesamten Aufnahmebereichs, insbesondere durch Schlitzscannen, durchgeführt. Nach diesem Untersuchungsvorgang wird das wenigstens eine Röntgenbild, insbesondere das bereits beschriebene Gesamtbild, analysiert, um den interessierenden Bereich zu lokalisieren. Für diesen interessierenden Bereich kann dann - bevorzugt mit derselben Röntgeneinrichtung - eine Tomosyntheseaufnahme dreidimensionaler Röntgendaten, bevorzugt unter Verwendung des spektral selektiven Röntgendetektors, erfolgen. Zwischen der Aufnahme der zweidimensionalen Röntgenbilder und der dreidimensionalen Röntgendaten kann der Patient in seiner Position verbleiben, wobei der Aufnahmestatus dem Patienten optional angezeigt werden kann. Zur Aufnahme der dreidimensionalen Röntgendaten durch Tomosynthese wird dann bevorzugt die Kollimierung hinsichtlich des lokalisierten interessierenden Bereichs angepasst. Beispielsweise nach einer Bestätigung durch den Benutzer kann dann der Tomosynthese-Scan erfolgen.

Dabei sei angemerkt, dass je nach interessierendem Bereich und der klinischen Fragestellung der interessierende Bereich durchaus dezentral bezüglich des vorangehenden zweidimensionalen Scans liegen kann. In Ausgestaltungen ist es auch denkbar, zusätzlich ein Kamerabild mit Skelettpunkten bereitzustellen, beispielsweise wenn eine wenigstens teilweise manuelle Markierung des interessierenden Bereichs erfolgen soll. Denkbar ist es jedoch auch, dass der interessierende Bereich für die Aufnahme der dreidimensionalen Röntgendaten vorab durch den Nutzer, beispielsweise anhand von Kameradaten, selektiert wurde.

Insgesamt in dieser zweiten Variante ist es denkbar, entweder nur die dreidimensionalen Röntgendaten als Eingangsdaten für den trainierten Auswertungsalgorithmus zu verwenden, bevorzugt ist es jedoch, sowohl die zweidimensionalen Röntgendaten als auch die dreidimensionalen Röntgendaten des interessierten Bereichs als Eingangsdaten zu verwenden.

In einer besonders bevorzugten, dritten Variante wird zunächst in einem ersten Schritt, wiederum bevorzugt durch Schlitzscannen, das wenigstens eine zweidimensionale Röntgenbild des Aufnahmebereichs aufgenommen, wobei wiederum insbesondere, wie oben beschrieben, ein Gesamtbild ermittelt werden kann. Jedoch erfolgt bereits während der Aufnahme des wenigstens einen zweidimensionalen Röntgenbilds des Aufnahmebereichs bevorzugt bildbasiert eine Überwachung, ob der interessierende Bereich, beispielsweise der Beckenbereich, im Aufnahmebereich erreicht wird. Ist dies der Fall, wird die Aufnahme des wenigstens einen Röntgenbildes abgebrochen und der Aufnahmemodus wird zur Aufnahme von dreidimensionalen Röntgendaten des interessierenden Bereichs, insbesondere mit dem spektral selektiven Röntgendetektor, angepasst. Hierbei wird wiederum bevorzugt ein Tomosynthese-Scan verwendet, um die dreidimensionalen Röntgendaten aufzunehmen. Mit anderen Worten wird in dieser Variante der zweidimensionale Scan automatisch angehalten, sobald der interessierende Bereich automatisch aufgefunden wird. In optionaler Ausgestaltung kann der interessierende Bereich einen Benutzer, beispielsweise zur Bestätigung oder Anpassung, angezeigt werden. Die Kollimierung wird bezüglich des interessierenden Bereichs angepasst und die dreidimensionalen Röntgendaten werden durch einen Tomosynthese-Scan aufgenommen. Besonders bevorzugt kann vorgesehen sein, ein Synthetikbild für den interessierenden Bereich zu ermitteln, insbesondere als synthetische Radiographie. Das Synthetikbild wird dann mit dem wenigstens einen insbesondere durch Schlitzscannen aufgenommenen zweidimensionalen Röntgenbild fusioniert. Sollte noch ein Anteil des Aufnahmebereichs fehlen, kann der 2D-Scan, also die Aufnahme des wenigstens einen Röntgenbilds, danach fortgesetzt werden. Auch hier kann, allgemein gesprochen, vorgesehen sein, als Eingangsdaten nur die dreidimensionalen Röntgendaten zu verwenden. Bevorzugt werden jedoch auch die zweidimensionalen Röntgendaten, insbesondere des Synthetikbilds, als Eingangsdaten für den trainierten Auswertungsalgorithmus verwendet. Selbstverständlich sind im Rahmen der Erfindung auch andere Varianten denkbar.

In einer besonders vorteilhaften Ausgestaltung der vorliegenden Erfindung kann vorgesehen sein, dass bei einer sich auf ein bestimmtes anatomisches Merkmal, insbesondere den Beckenkamm, beziehenden Knocheninformation der interessierende Bereich umfassend wenigstens ein Zusatzinformation bereitstellendes, zusätzliches, benachbartes anatomisches Merkmal, insbesondere die proximalen Femurepiphysen, gewählt wird. Bei Untersuchungen zum Einsatz künstlicher Intelligenz, aber auch hinsichtlich erfahrener Befundungsexperten, hat es sich gezeigt, dass auch andere anatomische Merkmale zweckmäßig in Betracht gezogen werden können, wenn die Knocheninformation ermittelt wird. Beispielsweise wird das Risser-Zeichen bezüglich des Darmbeinkamms (Beckenkamms), insbesondere der Darmbeinapophyse, definiert, wobei Radiologen häufig auch andere anatomische Merkmale in Betracht ziehen, wenn ein Risser-Score, also eine Kategorie des Risser-Zeichens, vergeben wird, beispielsweise die proximalen Femurepiphysen. Die trainierte Auswertungsfunktion kann auch dieses kognitive Verhalten des Radiologen nachahmen, wenn ihm hierzu die Möglichkeit gegeben wird, vorliegend indem gemäß dieser vorteilhaften Ausgestaltung der vorliegenden Erfindung ein gezielt größer als nur das der Definition zugrundeliegende anatomische Merkmal gewählter interessierender Bereich verwendet wird, der auch zusätzliche anatomische Merkmale enthält, die Zusatzinformationen liefern können. Dabei hat sich beim Trainieren von Auswertungsfunktionen bereits gezeigt, dass sich die Nutzung derartiger Zusatzinformationen nicht zwangsläufig nur auf vom manuellen Befunder bewusst in Betracht gezogene anatomische Merkmale bezieht, sondern auch andere Zusammenhänge, die sich gegebenenfalls dem Blick des Menschen entziehen können, erkannt werden, welche ebenso im Zusammenhang mit der Bewertung des für die Knocheninformation relevanten anatomischen Merkmals stehen. Insofern stellt eine derartige Erweiterung des interessierenden Bereichs auf benachbarte, zusätzliche anatomische Merkmale eine weitere Verbesserung der Genauigkeit und Verlässlichkeit bei der Bestimmung der Knocheninformation bereit.

Die vorliegende Erfindung nutzt künstliche Intelligenz im Rahmen der trainierten Auswertungsfunktion. Im Allgemeinen bildet eine trainierte Funktion kognitive Funktionen ab, die Menschen mit anderen menschlichen Gehirnen assoziieren. Durch Training basierend auf Trainingsdaten (Maschinenlernen) ist die trainierte Funktion in der Lage, sich an neue Umstände anzupassen und Muster zu detektieren und zu extrapolieren.

Allgemein gesagt können Parameter einer trainierten Funktion durch Training angepasst werden. Insbesondere können überwachtes Lernen, halbüberwachtes Lernen, nicht überwachtes Lernen, Reinforcement Learning und/oder aktives Lernen verwendet werden. Darüber hinaus kann auch Repräsentationslernen (auch als "feature learning" bekannt) eingesetzt werden. Die Parameter der trainierten Funktion können insbesondere iterativ durch mehrere Trainingsschritte angepasst werden.

Eine trainierte Funktion kann beispielsweise ein neuronales Netz, eine Support Vector Machine (SVM), einen Entscheidungsbaum und/oder ein Bayes-Netzwerk umfassen und/oder die trainierte Funktion kann auf k-means-Clustering, q-Learning, genetischen Algorithmen und/oder Zuordnungsregeln basieren. Insbesondere kann ein neuronales Netzwerk ein tiefes neuronales Netzwerk, ein Convolutional Neural Network (CNN) oder ein tiefes CNN sein. Darüber hinaus kann das neuronale Netzwerk ein Adversarial Network, ein tiefes Adversarial Network und/oder ein Generative Adversarial Network (GAN) sein.

Dabei wird im Rahmen der vorliegenden Erfindung bevorzugt eine trainierte Auswertungsfunktion benutzt, die ein Convolutional Neural Network (CNN) umfasst. In einer konkreten Ausgestaltung kann beispielsweise ein DenseNet verwendet werden. Ein DenseNet ist ein CNN, dass dichte Verbindungen zwischen Schichten verwendet, worin insbesondere alle Schichten mit passenden Eingangsdatendimensionen direkt miteinander verbunden werden.

Als Knocheninformation kann konkret ein Skelettreifemaß, insbesondere ein Risser-Zeichen bzw. eine Kategorie des Risser-Zeichens, oft auch als "Risser-Grade" bezeichnet, ermittelt werden. In diesem Fall wird also eine automatisierte Bildanalyse basierend auf Maschinenlernen vorgeschlagen, um die Skelettreife hinsichtlich des Risser-Zeichen-Klassifikationsschemas zu klassifizieren. Auch wenn das Risser-Zeichen als hauptsächliches Beispiel genannt wird, ist, wie gesagt, der Ansatz selbstverständlich auch auf andere Skelettreifemaße, beispielsweise SMS, übertragbar, da auch dort die Ausnutzung sowohl der Knochenmorphologie als auch des Knochenmaterialgehalts bzw. allgemein einer Knochendichteinformation von hohem Nutzen ist.

In einer Ausgestaltung der Erfindung kann ferner auch vorgesehen sein, dass das Verfahren ferner umfasst:

- Bereitstellen von Trainingsdatensätzen, jeweils umfassend spektrale Röntgendaten als Trainingseingangsdaten mit einer zugeordneten Trainingsknocheninformation,
- Trainieren einer Auswertungsfunktion mittels der Trainingsdatensätze und
- Bereitstellen der trainierten Auswertungsfunktion.

Ein alleinstehendes Trainingsverfahren ist im Rahmen der vorliegenden Erfindung selbstverständlich ebenso denkbar, dies ist jedoch hier nicht beansprucht. Die Auswertungsfunktion, insbesondere umfassend ein CNN, kann also trainiert werden, indem spektrale Röntgendaten, die insbesondere von menschlichen Experten hinsichtlich der Knocheninformation, insbesondere des Skelettreifemaßes, klassifiziert wurden, als Trainingsdaten herangezogen werden. Beispielsweise können durch menschliche Experten Risser-Zeichen-Kategorien, üblicherweise von 0 bis 5, den Trainingseingangsdaten zugeordnet werden, um Trainingsdatensätze zu ermitteln und zum Training zu verwenden. Im Rahmen der Inferenz wird dann nicht gesichteten spektralen Röntgendaten eine entsprechende Risser-Zeichen-Kategorie von 0 bis 5 zugeordnet. Dabei sei angemerkt, dass es selbstverständlich im Rahmen der vorliegenden Erfindung auch denkbar ist, als Ausgabe der trainierten Auswertungsfunktion Zwischenwerte zu verwenden, mithin nicht nur die üblichen Kategorien 0, 1, 2, 3, 4 und 5 zu erlauben, welche als keine Knochenbildung der Darmbein-Apophyse, < 25 % Knochenbildung, 25 - 50% Knochenbildung, 50 - 75 % Knochenbildung, 75 - 100 % Knochenbildung und komplette Knochenbildung der Apophyse definiert sind, sondern insbesondere kontinuierliche Werte für das Risser-Zeichen zu erlauben, beispielsweise 3,4.

Das beschriebene erfindungsgemäße Verfahren wird bevorzugt, insbesondere auch hinsichtlich der konkreten Möglichkeiten zur Aufnahme der Röntgendaten, durch die Steuereinrichtung einer Röntgeneinrichtung umgesetzt. Mithin betrifft die vorliegende Erfindung auch eine Röntgeneinrichtung, aufweisend eine einen Röntgenstrahler und einen insbesondere spektral selektiven Röntgendetektor umfassende Aufnahmeanordnung sowie eine zur Durchführung eines erfindungsgemäßen Verfahrens ausgebildete Steuereinrichtung. Sämtliche Ausführungen bezüglich des erfindungsgemäßen Verfahrens lassen sich analog auf die erfindungsgemäße Röntgeneinrichtung übertragen, mit welcher mithin ebenso die bereits genannten Vorteile erhalten werden können.

Bei dem spektral selektiven Röntgendetektor handelt es sich dabei mit besonderem Vorteil um einen 2-Schicht-Detektor oder einen photonenzählenden Detektor. Bei der Röntgeneinrichtung kann es sich um eine zwei Roboterarme umfassende Röntgeneinrichtung handeln, wobei sowohl der Röntgenstrahler als auch der Röntgendetektor an einem Roboterarm angeordnet sein können, um verschiedene Aufnahmegeometrien einzustellen, beispielsweise um eine Tomosynthese des interessierenden Bereichs zu ermöglichen. Die Röntgeneinrichtung weist ferner bevorzugt einen Kollimator auf, beispielsweise zur Realisierung eines Schlitzscannens. Der Aufnahmebetrieb wird von der Steuereinrichtung gesteuert. Diese ist auch ausgebildet, die trainierte Auswertungsfunktion einzusetzen, um unmittelbar mit aufgenommenen Röntgendaten auch die Knocheninformation liefern zu können. In der Steuereinrichtung können beispielsweise auf unterschiedliche Anwendungsfälle ausgerichtete Untersuchungsprogramme gespeichert sein, die eine konkrete Anwendung des erfindungsgemäßen Verfahrens auf unterschiedliche klinische Fragestellungen erlauben. So kann beispielsweise hinsichtlich einer Untersuchung bei Skoliose das Untersuchungsprogramm so ausgestaltet sein, dass ein 2D-Scan in einem die Wirbelsäule betreffenden Aufnahmebereich erfolgt, wobei bevorzugt zusätzlich in einem den Beckenbereich betreffenden interessierenden Bereich ein 3D-Scan, insbesondere eine Tomosynthese, erfolgen kann, um dreidimensionale Röntgendaten als Eingangsdaten für die Auswertungsfunktion zu bestimmen, welche dann beispielsweise eine Risser-Zeichen-Kategorie als Skelettreifemaß ausgeben kann. Dabei ist eine Umsetzung nach Art der oben beschriebenen dritten Variante besonders bevorzugt.

Um das erfindungsgemäße Verfahren durchführen zu können, kann die Röntgeneinrichtung insbesondere eine allgemeiner definierte erfindungsgemäße Ermittlungseinrichtung zur Ermittlung einer die Skelettreife eines Patienten beschreibenden Knocheninformation umfassen. Diese weist auf:

- eine erste Schnittstelle zur Bereitstellung von Eingangsdaten, umfassend spektral aufgelöste Röntgendaten eines zur Beurteilung der Skelettreife heranzuziehenden interessierenden Bereichs des Patienten mit wenigstens zwei auf unterschiedliche Röntgenspektren bezogenen Röntgenbilddatensätzen,
- eine Auswertungseinheit zum Anwenden einer trainierten Auswertungsfunktion auf die Eingangsdaten zum Erhalt der Knocheninformation der Ausgangsdaten, und
- eine zweite Schnittstelle zur Ausgabe der Knocheninformation

Die Röntgendaten werden wenigstens teilweise als Teil oder im Rahmen eines einen größeren Aufnahmebereichs als den interessierenden Bereich betreffenden Untersuchungsvorgangs aufgenommen, und wenigstens ein zweidimensional aufgenommenes Röntgenbild des Untersuchungsvorgangs und/oder eine empfangene Nutzerinformation zur Lokalisierung des interessierend Bereichs wird ausgewertet, wobei das Auswertungsergebnis zur nachfolgenden oder unterbrechenden Aufnahme dreidimensionaler, nur auf den interessierend Bereich bezogener dreidimensionaler Röntgendaten und/oder zur Auswahl von Eingangdaten zu verwendenden zweidimensionalen Röntgendaten der Röntgenbilder werwendet wird.

Auch auf die Ermittlungseinrichtung lassen sich sämtliche Ausführungen bezüglich der Röntgeneinrichtung und des Verfahrens analog übertragen. Bezogen auf die Steuereinrichtung der Röntgeneinrichtung kann diese zusätzlich noch eine Aufnahmeeinheit zur Steuerung des Aufnahmebetriebs aufweisen, welche den Aufnahmebetrieb konkret steuert und bei Vorhandensein von die Aufnahme betreffenden Schritten der konkreten Ausgestaltung des erfindungsgemäßen Verfahrens auch Teil der Ermittlungseinrichtung sein kann.

Ein erfindungsgemäßes Computerprogramm ist direkt in ein Speichermittel einer erfindungsgemäßen Ermittlungseinrichtung bzw. einer Steuereinrichtung einer erfindungsgemäßen Röntgeneinrichtung ladbar und weist Programmmittel auf, um die Schritte eines erfindungsgemäßen Verfahrens durchzuführen, wenn das Computerprogramm auf der Recheneinrichtung, insbesondere Ermittlungseinrichtung bzw. Steuereinrichtung, ausgeführt wird. Die Steuereinrichtung bzw. die Ermittlungseinrichtung kann hierzu einen Prozessor umfassen. Das Computerprogramm kann auf einem elektronisch lesbaren Datenträger gemäß der vorliegenden Erfindung gespeichert sein, welcher mithin darauf gespeicherte Steuerinformationen umfasst, welche wenigstens ein erfindungsgemäßes Computerprogramm umfassen und bei Verwendung des Datenträgers in einer Recheneinrichtung diese ausbilden, die Schritte eines erfindungsgemäßen Verfahrens durchzuführen. Bei dem Datenträger kann es sich insbesondere um einen nichttransienten Datenträger, beispielsweise eine CD-ROM, handeln.

Weitere Vorteile und Einzelheiten der vorliegenden Erfindung ergeben sich aus den im Folgenden beschriebenen Ausführungsbeispielen sowie anhand der Zeichnung. Dabei zeigen:

Fig. 1     ein Ausführungsbeispiel eines künstlichen neuronalen Netzes,

Fig. 2     ein Ausführungsbeispiel eines Convolutional Neural Network,

Fig. 3     einen Ablaufplan einer ersten Ausgestaltung zur Bereitstellung von Eingangsdaten,

Fig. 4     einen Ablaufplan eines zweiten Ausführungsbeispiels zur Bereitstellung von Eingangsdaten,

Fig. 5     einen Ablaufplan eines dritten Ausführungsbeispiels zur Bereitstellung von Eingangsdaten,

Fig. 6     einen Ablaufplan zur Ermittlung einer Knocheninformation,

Fig. 7     eine Ansicht des Beckenbereichs eines Menschen,

Fig. 8     eine Skizze zur Erläuterung von Kategorien des Risser-Zeichens,

Fig. 9     eine Prinzipskizze einer erfindungsgemäßen Röntgeneinrichtung, und

Fig. 10    die funktionale Struktur einer Steuereinrichtung der Röntgeneinrichtung.

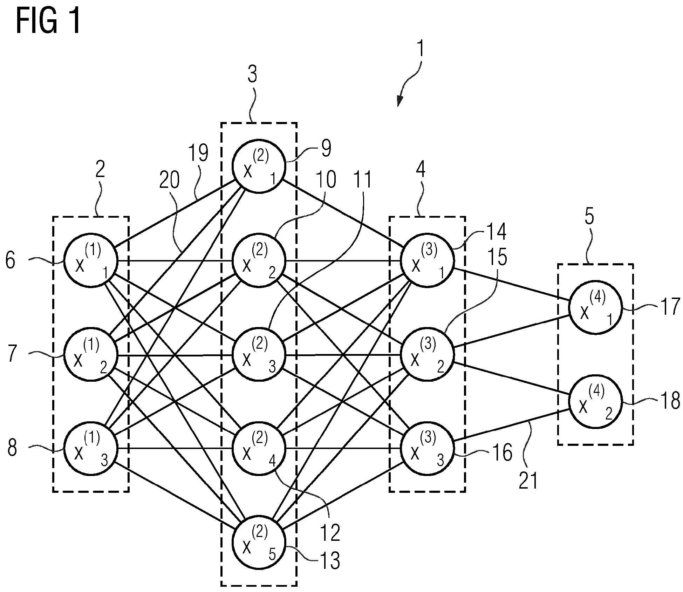

Fig. 1 zeigt ein Ausführungsbeispiel eines künstlichen neuronalen Netzes 1. Englische Ausdrücke für das künstliche neuronale Netz 1 sind "artificial neural network", "neural network", "artificial neural net" oder "neural net".

Das künstliche neuronale Netzwerk 1 umfasst Knoten 6 bis 18 (nodes) und Kanten 19 bis 21 (edges), wobei jede Kante 19 bis 21 eine gerichtete Verbindung von einem ersten Knoten 6 bis 18 zu einem zweiten Knoten 6 bis 18 ist. Im Allgemeinen sind der erste Knoten 6 bis 18 und der zweite Knoten 6 bis 18 unterschiedliche Knoten 6 bis 18, es ist jedoch auch denkbar, dass der erste Knoten 6 bis 18 und der zweite Knoten 6 bis 18 identisch sind. Beispielsweise ist in Fig. 1 die Kante 19 eine gerichtete Verbindung von dem Knoten 6 zu dem Knoten 9 und die Kante 21 ist eine gerichtete Verbindung von dem Knoten 16 zu dem Knoten 18. Eine Kante 19 bis 21 von einem ersten Knoten 6 bis 18 zu einem zweiten Knoten 6 bis 18 wird als eingehende Kante ("ingoing edge") für den zweiten Knoten 6 bis 18 und als ausgehende Kante ("outgoing edge") für den ersten Knoten 6 bis 18 bezeichnet.

In diesem Ausführungsbeispiel können die Knoten 6 bis 18 des künstlichen neuronalen Netzes 1 in Schichten 2 bis 5 (layers) angeordnet werden, wobei die Schichten eine intrinsische Ordnung aufweisen können, die durch die Kanten 19 bis 21 zwischen den Knoten 6 bis 18 eingeführt wird. Insbesondere können Kanten 19 bis 21 nur zwischen benachbarten Schichten von Knoten 6 bis 18 vorgesehen sein. Im dargestellten Ausführungsbeispiel existiert eine Eingabeschicht 2, die lediglich die Knoten 6, 7, 8 aufweist, jeweils ohne eingehende Kante. Die Ausgangsschicht 5 umfasst nur die Knoten 17, 18, jeweils ohne ausgehende Kanten, wobei ferner versteckte Schichten 3 und 4 zwischen der Eingangsschicht 2 und der Ausgangsschicht 5 liegen. Im Allgemeinen Fall kann die Zahl der versteckten Schichten 3, 4 beliebig gewählt werden. Die Zahl der Knoten 6, 7, 8 der Eingangsschicht 2 entspricht üblicherweise der Zahl der Eingabewerte in das neuronale Netzwerk 1, und die Zahl der Knoten 17, 18 in der Ausgangsschicht 5 entspricht üblicherweise der Zahl der Ausgabewerte des neuronalen Netzwerks 1.

Insbesondere kann eine (reale) Zahl den Knoten 6 bis 18 des neuronalen Netzwerks 1 zugeordnet werden. Dabei bezeichnet $x^{(n)}_i$ den Wert des i-ten Knotens 6 bis 18 der n-ten Schicht 2 bis 5. Die Werte der Knoten 6, 7, 8 der Eingabeschicht 2 sind äquivalent zu den Eingabewerten des neuronalen Netzwerks 1, während die Werte der Knoten 17, 18 der Ausgangsschicht 5 äquivalent zu den Ausgabewerten des neuronalen Netzwerks 1 sind. Darüber hinaus kann jeder Kante 19, 20, 21 ein Gewicht in Form einer realen Zahl zugeordnet sein. Insbesondere ist das Gewicht eine reale Zahl im Intervall [-1, 1] oder im Intervall [0, 1,]. Dabei bezeichnet $w^{(m,n)}_{i,j}$ das Gewicht der Kante zwischen den i-ten Knoten 6 bis 18 der m-ten Schicht 2 bis 5 und den j-ten Knoten 6 bis 18 der n-ten Schicht 2 bis 5. Ferner wird die Abkürzung $w^{(n)}_{i,j}$ für das Gewicht $w^{(n,n+1)}_{i,j}$ definiert.

Um Ausgangswerte des neuronalen Netzes 1 zu berechnen, werden die Eingangswerte durch das neuronale Netz 1 propagiert. Insbesondere können die Werte der Knoten 6 bis 18 des (n+1)-ten Schicht 2 bis 5 basierend auf den Werten der Knoten 6 bis 18 der n-ten Schicht 2 bis 5 berechnet werden durch

$$x^{(n+1)}_j \;=\; f\left(\sum_i x^{(n)}_i \cdot w^{(n)}_{i,j}\right).$$

Dabei ist f eine Transferfunktion, die auch als Aktivierungsfunktion bezeichnet werden kann. Bekannte Transferfunktionen sind Stufenfunktionen, Sigmoidfunktionen (beispielsweise die logistische Funktion, die verallgemeinerte logistische Funktion, der Tangens hyperbolicus, der Arkustangens, die Fehlerfunktion, die Smoothstep-Funktion) oder Gleichrichterfunktionen (Rectifier). Die Transferfunktion wird im Wesentlichen für Normierungszwecke verwendet.

Insbesondere werden die Werte schichtweise durch das neuronale Netz 1 propagiert, wobei Werte der Eingangsschicht 2 durch die Eingangsdaten des neuronalen Netzes 1 gegeben sind. Werte der ersten versteckten Schicht 3 können basierend auf den Werten der Eingangsschicht 2 des neuronalen Netzes 1 berechnet werden, Werte der zweiten versteckten Schicht 4 können basierend auf den Werten in der ersten versteckten Schicht 3 berechnet werden usw.

Um die Werte $w^{(n)}_{i,j}$ für die Kanten 19 bis 21 festlegen zu können, muss das neuronale Netz 1 unter Verwendung von Trainingsdaten trainiert werden. Insbesondere umfassen Trainingsdaten Trainingseingangsdaten und Trainingsausgangsdaten, die im Folgenden als $t_i$ bezeichnet werden. Für einen Trainingsschritt wird das neuronale Netzwerk 1 auf die Trainingseingangsdaten angewendet, um berechnete Ausgangsdaten zu ermitteln. Insbesondere umfassen die Trainingsausgangsdaten und die berechneten Ausgangsdaten eine Zahl von Werten, wobei sich die Zahl als die Zahl der Knoten 17, 18 der Ausgangsschicht 5 bestimmt.

Insbesondere wird ein Vergleich zwischen den berechneten Ausgangsdaten und den Trainingsausgangsdaten verwendet, um die Gewichte innerhalb des neuronalen Netzes 1 rekursiv anzupassen (Rückpropagierungsalgorithmus - "back propagation algorithm"). Insbesondere können die Gewichte entsprechend

$$w'^{(n)}_{i,j} \;=\; w^{(n)}_{i,j} \;-\; \gamma \cdot \delta^{(n)}_j \cdot x^{(n)}_i$$

geändert werden, wobei $\gamma$ eine Lernrate ist und die Zahlen $\delta^{(n)}_j$ rekursiv berechnet werden können als

$$\delta^{(n)}_j \;=\; \left(\sum_k \delta^{(n+1)}_k \cdot w^{(n+1)}_{j,k}\right) \cdot f'\left(\sum_i x^{(n)}_i \cdot w^{(n)}_{i,j}\right)$$

basierend auf $\delta^{(n+1)}_j$, wenn die (n+1)-te Schicht nicht die Ausgangsschicht 5 ist, und

$$\delta^{(n)}_j \;=\; \left(x^{(n+1)}_k - t^{(n-1)}_j\right) \cdot f'\left(\sum_i x^{(n)}_i \cdot w^{(n)}_{i,j}\right)$$

falls die (n+1)-te Schicht die Ausgangsschicht 5 ist, wobei f' die erste Ableitung der Aktivierungsfunktion ist und $y_j^{(n+1)}$ der Vergleichstrainingswert für den j-ten Knoten 17, 18 der Ausgangsschicht 5 ist.

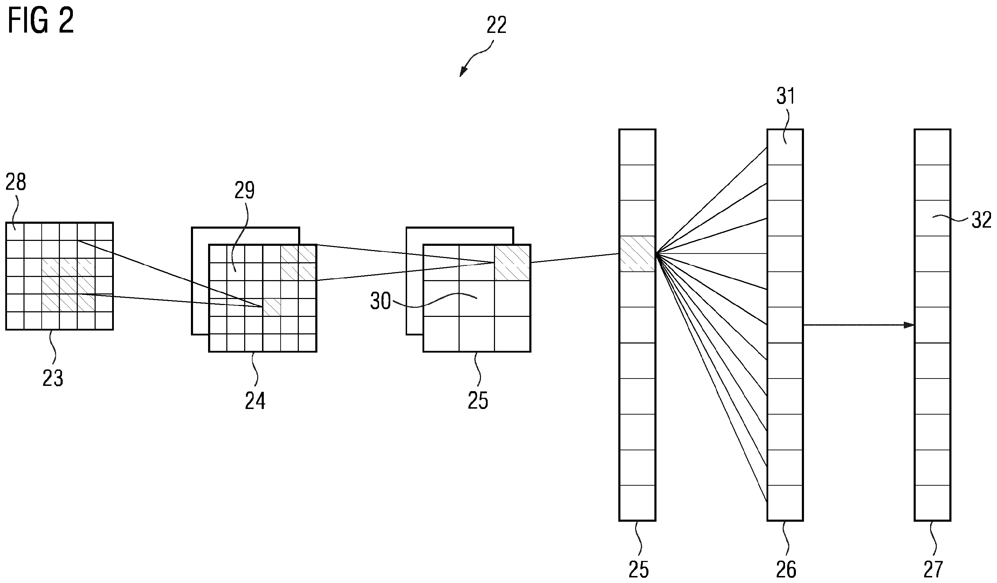

Im Folgenden wird im Hinblick auf Fig. 2 auch ein Beispiel für ein Convolutional Neural Network (CNN) gegeben. Dabei ist zu beachten, dass der Ausdruck "Schicht" ("layer") dort auf leicht andere Art und Weise als für klassische neuronale Netze eingesetzt wird. Für ein klassisches neuronales Netz verweist der Ausdruck "Schicht" nur auf den Satz von Knoten, der eine Schicht bildet, mithin eine bestimmte Generation von Knoten. Für ein Convolutional Neural Network, wird der Ausdruck "Schicht" oft als ein Objekt benutzt, das aktiv Daten verändert, in anderen Worten als ein Satz von Knoten derselben Generation und entweder den Satz eingehender oder ausgehender Kanten.

Fig. 2 zeigt ein Ausführungsbeispiel eines Convolutional Neural Network 22. In dem dargestellten Ausführungsbeispiel umfasst das Convolutional Neural Network 22 eine Eingangsschicht 23, eine Faltungsschicht 24 (Convolutional Layer), eine Pooling-Schicht 25, eine vollständig verbundene Schicht 26 und eine Ausgangsschicht 27. In alternativen Ausgestaltungen kann das Convolutional Neural Network 22 mehrere Faltungsschichten 24, mehrere Pooling-Schichten 25 und mehrere vollständig verbundene Schichten 26, genau wie andere Arten von Schichten, enthalten. Die Reihenfolge der Schichten kann beliebig gewählt werden, wobei üblicherweise vollständig verbundene Schichten 26 die letzten Schichten vor der Ausgangsschicht 27 bilden.

Insbesondere können innerhalb eines Convolutional Neural Network 22 die Knoten 28 bis 32 einer der Schichten 23 bis 27 als in einer d-dimensionalen Matrix oder als d-dimensionales Bild angeordnet verstanden werden. Insbesondere kann im zweidimensionalen Fall der Wert eines Knotens 28 bis 32 mit den Indizes i, j in der n-ten Schicht 23 bis 27 als $x^{(n)}[i,j]$ bezeichnet werden. Es ist darauf hinzuweisen, dass die Anordnung der Knoten 28 bis 31 einer Schicht 23 bis 27 keinerlei Auswirkung auf die Berechnungen innerhalb des Convolutional Neural Network 22 als solches hat, da diese Auswirkungen ausschließlich durch die Struktur und die Gewichte der Kanten gegeben sind.

Eine Faltungsschicht 24 ist insbesondere dadurch ausgezeichnet, dass die Struktur und die Gewichte der eingehenden Kanten eine Faltungsoperation basierend auf einer bestimmten Zahl von Kernen bildet. Insbesondere können die Struktur und die Gewichte der eingehenden Kanten so gewählt werden, dass die Werte $x_k^{(n)}$ der Knoten 29 der Faltungsschicht 24 als eine Faltung $x_k^{(n)} = K_k * x^{(n-1)}$ basierend auf den Werten $x^{(n-1)}$ der Knoten 28 der vorangehenden Schicht 23 ermittelt werden, wobei die Faltung * im zweidimensionalen Fall definiert werden kann als

$$x_k^{(n)}[i,j] = (K_k * x^{(n-1)})[i,j] = \sum_{i'} \sum_{j'} K_k[i',j'] \cdot x^{(n-1)}[i-i', \; j-j'].$$

Darin ist der k-te Kern $K_k$ eine d-dimensionale Matrix, in diesem Ausführungsbeispiel eine zweidimensionale Matrix, die üblicherweise klein im Vergleich zur Zahl der Knoten 28 bis 32 ist, beispielsweise eine 3x3-Matrix oder eine 5x5-Matrix. Insbesondere impliziert dies, dass die Gewichte der eingehenden Kanten nicht unabhängig sind, sondern so gewählt sind, dass sie obige Faltungsgleichung erzeugen. Im Beispiel für einen Kern, der eine 3x3-Matrix bildet, existieren nur neun unabhängige Gewichte (wobei jeder Eintrag der Kern-Matrix einem unabhängigen Gewicht entspricht), ungeachtet der Zahl der Knoten 28 bis 32 in der entsprechenden Schicht 23 bis 27. Insbesondere ist für eine Faltungsschicht 24 die Zahl der Knoten 29 in der Faltungsschicht 24 äquivalent der Zahl der Knoten 28 in der vorangehenden Schicht 23 multipliziert mit der Zahl der Faltungskerne.

Wenn die Knoten 28 der vorangehenden Schicht 23 als eine d-dimensionale Matrix angeordnet sind, kann die Nutzung der Mehrzahl von Kernen als Hinzufügung einer weiteren Dimension, die auch als Tiefendimension bezeichnet wird, verstanden werden, so dass die Knoten 29 der Faltungsschicht 24 als eine (d+1)-dimensionale Matrix angeordnet sind. Wenn die Knoten 28 der vorangehenden Schicht 23 bereits als eine (d+1)-dimensionale Matrix mit einer Tiefendimension angeordnet sind, kann die Nutzung einer Mehrzahl von Faltungskernen als Expansion entlang der Tiefendimension verstanden werden, so dass die Knoten 29 der Faltungsschicht 24 gleichermaßen als eine (d+1)-dimensionale Matrix angeordnet sind, wobei die Größe der (d+1)-dimensionalen Matrix in der Tiefendimension um den durch die Zahl der Kerne gebildeten Faktor größer ist als in der vorangehenden Schicht 23.

Der Vorteil der Nutzung von Faltungsschichten 24 ist, dass die räumlich lokale Korrelation der Eingangsdaten ausgenutzt werden kann, indem ein lokales Verbindungsmuster zwischen Knoten benachbarter Schichten geschaffen wird, insbesondere dadurch, dass jeder Knoten nur zu einem kleinen Bereich der Knoten der vorangehenden Schicht Verbindungen aufweist.

Im dargestellten Ausführungsbeispiel umfasst die Eingangsschicht 23 sechsunddreißig Knoten 28, die als eine zweidimensionale 6x6-Matrix angeordnet sind. Die Faltungsschicht 24 umfasst zweiundsiebzig Knoten 29, die als zwei zweidimensionale 6x6-Matrizen angeordnet sind, wobei jede der beiden Matrizen das Ergebnis einer Faltung der Werte der Eingangsschicht 23 mit einem Faltungskern ist. In gleicher Weise können die Knoten 29 der Faltungsschicht 24 als in einer dreidimensionalen 6x6x2-Matrix angeordnet verstanden werden, wobei die zuletzt genannte Dimension die Tiefendimension ist.

Eine Pooling-Schicht 25 zeichnet sich dadurch aus, dass die Struktur und die Gewichte der eingehenden Kanten sowie die Aktivierungsfunktion ihrer Knoten 30 eine Pooling-Operation basierend auf einer nichtlinearen Pooling-Funktion f definieren. Beispielsweise können im zweidimensionalen Fall die Werte $x^{(n)}$ der Knoten 30 der Pooling-Schicht 25 basierend auf den Werten $x^{(n+1)}$ der Knoten 29 der vorangehenden Schicht 24 als

$$x^{(n)}[i,j] = f(x^{(n-1)}[id_1, jd_2], …, x^{(n-1)}[id_1+d_1-1, jd_2+d_2-1])$$

berechnet werden. In anderen Worten kann durch die Verwendung einer Pooling-Schicht 25 die Zahl der Knoten 29, 30 reduziert werden, indem eine Anzahl von $d_1 \times d_2$ benachbarter Knoten 29 in der vorangehenden Schicht 24 durch einen einzelnen Knoten 30 ersetzt werden, der als eine Funktion der Werte der genannten Anzahl benachbarter Knoten 29 berechnet wird. Insbesondere kann die Pooling-Funktion f eine Maximumsfunktion, eine Durchschnittsbildung oder die L2-Norm sein. Insbesondere können für eine Pooling-Schicht 25 die Gewichte der eingehenden Kanten festgelegt sein und nicht durch Training modifiziert sein.

Der Vorteil der Verwendung einer Pooling-Schicht 25 ist, dass die Zahl der Knoten 29, 30 und die Zahl der Parameter reduziert wird. Dies führt zu einer Reduktion der notwendigen Berechnungsmenge innerhalb des Convolutional Neural Network 22 und somit zu einer Steuerung der Überanpassung.

Im dargestellten Ausführungsbeispiel handelt es sich bei der Pooling-Schicht 25 um eine Max-Pooling-Schicht, in der vier benachbarte Knoten mit nur einem einzigen Knoten ersetzt werden, dessen Wert durch das Maximum der Werte der vier benachbarten Knoten gebildet wird. Das Max-Pooling wird auf jede d-dimensionale Matrix der vorherigen Schicht angewendet; in diesem Ausführungsbeispiel wird das Max-Pooling auf jede der zwei zweidimensionalen Matrizen angewendet, so dass sich die Zahl der Knoten von zweiundsiebzig auf achtzehn reduziert.

Eine vollständig verbundene Schicht 26 zeichnet sich dadurch aus, dass eine Mehrzahl, insbesondere alle, Kanten zwischen den Knoten 30 der vorherigen Schicht 25 und den Knoten 31 der vollständig verbundenen Schicht 26 vorhanden sind, wobei das Gewicht jeder der Kanten individuell angepasst werden kann. In diesem Ausführungsbeispiel werden die Knoten 30 der vorangehenden Schicht 25 und der vollständig verbundenen Schicht 26 sowohl als zweidimensionale Matrizen als auch als nichtzusammenhängende Knoten (dargestellt als eine Zeile von Knoten, wobei die Zahl der Knoten zur besseren Darstellbarkeit reduziert wurde) gezeigt. In diesem Ausführungsbeispiel ist die Zahl der Knoten 31 in der vollständig verbundenen Schicht 26 gleich der Anzahl der Knoten 30 in der vorangehenden Schicht 25. In alternativen Ausführungsformen kann die Zahl der Knoten 30, 31 unterschiedlich sein.

Darüber hinaus werden in diesem Ausführungsbeispiel die Werte der Knoten 32 der Ausgangsschicht 27 bestimmt, indem die Softmax-Funktion auf die Werte der Knoten 31 der vorangehenden Schicht 26 angewendet wird. Durch Anwendung der Softmax-Funktion ist die Summe der Werte aller Knoten 32 der Ausgangsschicht 27 eins und alle Werte aller Knoten 32 der Ausgangsschicht sind reale Zahlen zwischen 0 und **1**. Wenn das Convolutional Neural Network 22 zur Klassifizierung von Eingangsdaten genutzt wird, können insbesondere die Werte der Ausgangsschicht 27 als Wahrscheinlichkeit dafür interpretiert werden, dass die Eingangsdaten in eine der unterschiedlichen Klassen fallen.

Ein Convolutional Neural Network 22 kann ebenso eine ReLU-Schicht aufweisen, wobei ReLU als Akronym für "rectified linear units" steht. Insbesondere ist die Zahl der Knoten und die Struktur der Knoten innerhalb einer ReLU-Schicht äquivalent zu der Zahl der Knoten und der Strukturen der Knoten der vorangehenden Schicht. Der Wert jedes Knotens in der ReLU-Schicht kann insbesondere durch Anwendung einer Gleichrichtungsfunktion (rectifier function) auf den Wert des entsprechenden Knoten der vorangehenden Schicht berechnet werden. Beispiele für Gleichrichterfunktionen sind $f(x)=\max(0,x)$, der Tangens hyperbolicus oder die Sigmoidfunktion.

Convolutional Neural Networks 22 können insbesondere basierend auf den Rückpropagierungsalgorithmus trainiert werden. Um eine Überanpassung (overfitting) zu vermeiden, können Verfahren der Regularisierung eingesetzt werden, beispielsweise Dropout einzelner Knoten 28 bis 32, stochastisches Pooling, Nutzen von künstlichen Daten, Gewichtszerfall basierend auf der L1- oder der L2-Norm oder Maximalnorm-Einschränkungen. Im Folgenden wird nun die vorliegende Erfindung anhand von mehreren Ausführungsbeispielen erläutert, wobei hier rein beispielhaft eine automatische Ermittlung einer Kategorie des Risser-Zeichens als Skelettreifemaß beschrieben wird. Das bedeutet, der interessierende Bereich ist der Beckenbereich, insbesondere umfassend das Darmbein als hauptsächlich relevantes, das Risser-Zeichen definierendes Merkmal, dass die dortige Knochenbildung (Ossifikation) anzeigt. Dabei erfolgt hier die Aufnahme von als Eingangsdaten für eine trainierte Auswertungsfunktion, die die Kategorie des Risser-Zeichens ermittelt, nutzbaren zwei- oder dreidimensionalen Röntgendaten im Rahmen eines Untersuchungsvorgangs zur Skoliose, durch den auch die Wirbelsäule in einem diesen umfassenden Aufnahmebereich abgebildet werden soll. Verwendet wird eine Röntgeneinrichtung mit einem bewegbaren Röntgenstrahler und einem bewegbaren spektral selektiven Röntgendetektor. Der spektral selektive Röntgendetektor ist beispielhaft als Zwei-Schicht-Detektor ausgebildet.

Allgemein gilt für alle im Folgenden beschriebenen Ausführungsbeispiele, dass zunächst in einem Schritt S1 zumindest begonnen wird, wenigstens ein zweidimensionales Röntgenbild des gesamten Aufnahmebereichs, hier in einer frontalen Bildebene, aufzunehmen. Dabei können bereits spektrale Röntgenbilder, die mithin Röntgendaten für unterschiedliche Energiespektren umfassen, aufgenommen werden. Die Aufnahme der Röntgenbilder erfolgt durch Schlitzscannen (Slot-Scanning), um Streustrahlungseffekte zu reduzieren und geometrische Verzerrungen möglichst weitgehend zu vermeiden. Im konkreten Ausführungsbeispiel können beispielsweise drei solcher Röntgenbilder, die zu einem Gesamtbild zusammengesetzt werden, aufgenommen werden, einmal eines vom oberen Rücken- und Nackenbereich, eines vom mittleren Torsobereich und eines vom Beckenbereich.

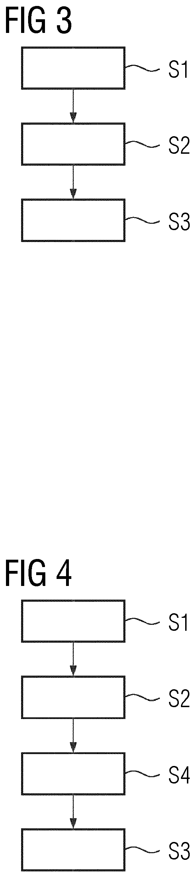

In der ersten hier dargestellten Variante gemäß Fig. 3 wird zunächst im Schritt S1 das Gesamtbild vollständig aufgenommen, bevor in einem Schritt S2 darin automatisch bildbasiert eine Lokalisierung des interessierenden Bereichs, also des Beckenbereichs, erfolgt. Im Schritt S3 werden die Eingangsdaten zusammengestellt, indem als zweidimensionale spektrale Bilddaten die in dem interessierenden Bereich gemäß der Lokalisierungsinformation des Schrittes S2 liegenden spektralen Röntgendaten des Gesamtbilds, umfassend mehrere zweidimensionale Röntgenbilddatensätze für die unterschiedlichen Energiespektren, ausgewählt werden. Im Schritt S3 wird zudem ein Kombinationsdatensatz als weiterer zweidimensionaler Röntgenbilddatensatz gebildet, der, wie aus der XDA bekannt, sich auf den Knochenmineralgehalt im interessierenden Bereich bezieht, mithin Knochenmineralien hervorhebt. Die Eingangsdaten umfassen in diesem Fall also einen zweidimensionalen Hochenergie-Röntgenbilddatensatz des interessierenden Bereichs, einen zweidimensionalen Niedrigenergie-Röntgenbilddatensatz des interessierenden Bereichs sowie den zweidimensionalen Kombinationsbilddatensatz, der durch gewichtete Linearkombination aus dem Hochenergie-Röntgenbilddatensatz und dem Niedrigenergie-Röntgenbilddatensatz gewonnen wurde.

Eine zweite Variante stellt das Ausführungsbeispiel gemäß Fig. 4 dar, in dem wiederum zunächst der 2D-Scan des Aufnahmebereichs vollständig im Schritt S1 durchgeführt wurde, sodass das Gesamtbild vorliegt. Ebenso wie im ersten Ausführungsbeispiel gemäß Fig. 3 erfolgt im Schritt S2 die Lokalisierung des interessierenden Bereichs, bevorzugt wiederum bildbasiert auf dem Gesamtbild. Nachdem dieser, beispielsweise durch einen Nutzer, bestätigt wurde, wird der Aufnahmemodus der Röntgeneinrichtung geändert und es erfolgt im Schritt S4 ein 3D-Scan des interessierenden Bereichs, hier des Beckenbereichs. Dabei wird eine Tomosynthese durchgeführt, um dreidimensionale spektrale Röntgendaten aufzunehmen, die mithin einen dreidimensionalen Hochenergie-Röntgenbilddatensatz und einen dreidimensionalen Niedrigenergie-Röntgenbilddatensatz umfassen. Dabei sei angemerkt, dass der interessierende Bereich allgemein selbstverständlich dezentral bezüglich des Aufnahmebereichs liegen kann, abhängig von dem interessierenden Bereich und der klinischen Fragestellung.

Im Schritt S3 werden dann wiederum die Eingangsdaten zusammengestellt. In dieser zweiten Variante wird sowohl für die zweidimensionalen spektralen Röntgendaten des Gesamtbilds im interessierenden Bereich als auch für die dreidimensionalen Röntgendaten des 3D-Scans ein Kombinationsdatensatz, der den Knochenmineralgehalt hervorhebt, ermittelt. Die Eingangsdaten werden gebildet durch den zweidimensionalen Hochenergie-Röntgenbilddatensatz, den zweidimensionalen Niedrigenergie-Röntgenbilddatensatz, den zweidimensionalen Kombinationsbilddatensatz, den dreidimensionalen Hochenergie-Röntgenbilddatensatz, den dreidimensionalen Niedrigenergie-Röntgenbilddatensatz und den dreidimensionalen Kombinationsbilddatensatz. Es ist auch denkbar, nur auf den dreidimensionalen spektralen Röntgendaten zu arbeiten.

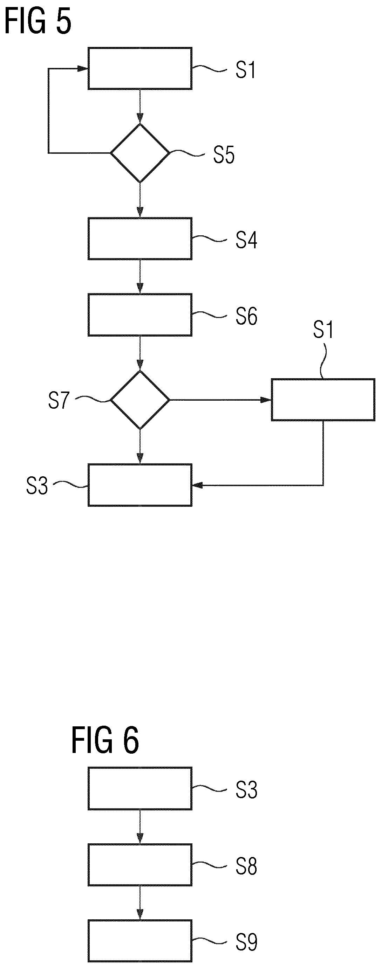

In einem besonders bevorzugten dritten Ausführungsbeispiel gemäß Fig. 5 wird auch zunächst mit dem 2D-Scan des Aufnahmebereichs begonnen. Jedoch wird bereits während des Aufnahmebetriebs im Schritt S5 ständig überwacht, ob der interessierende Bereich erreicht ist. Dies geschieht durch Bildauswertung der im 2D-Scan aufgenommenen Röntgenbilder. Solange der interessierende Bereich nicht erreicht ist, wird mit Schritt S1 fortgefahren.

Ist jedoch im Schritt S5 der interessierende Bereich erreicht, hier also der Beckenbereich, wird der 2D-Scan des Aufnahmebereichs abgebrochen und es kann optional eine Nachricht an einen Nutzer ausgegeben werden, der dann das Erreichen des interessierenden Bereichs bestätigen kann. Der Aufnahmemodus wird dann wiederum, genau wie die Kollimierung, geändert, um im Schritt S4 einen 3D-Scan des interessierenden Bereichs aufzunehmen, wie in der zweiten Variante als Tomosynthese-Scan. Ist der Tomosynthese-Scan abgeschlossen, liegen also die dreidimensionalen spektralen Röntgendaten vor, werden diese in einem Schritt S6 genutzt, um eine synthetische Radiographie des interessierenden Bereichs als zweidimensionales Synthetikbild zu ermitteln. Dieses Synthetikbild wird dann mit den in Schritt S1 aufgenommenen zweidimensionalen Röntgenbildern fusioniert, sodass eine Aufnahme im interessierenden Bereich nicht erneut erfolgen muss und somit Röntgendosis eingespart werden kann.

In einem Schritt S7 wird dann überprüft, ob der komplette Aufnahmebereich im bisherigen 2D-Scan und durch das Synthetikbild abgebildet ist, was bei einem die Wirbelsäule betreffenden Aufnahmebereich mit dem Beckenbereich als interessierender Bereich dann, wenn von oben begonnen wurde, der Fall sein sollte. Ist dies jedoch nicht der Fall, wird der 2D-Scan des Schrittes S1 zunächst fortgesetzt, bevor im Schritt S3 die Eingangsdaten zusammengestellt werden. Wie bezüglich der zweiten Variante dargelegt, umfassen die Eingangsdaten dann bevorzugt die dreidimensionalen spektralen Röntgendaten des Gesamtbilds sowie den zweidimensionalen Kombinationsdatensatz wie auch die dreidimensionalen Röntgendaten des Tomosynthese-Scans gemeinsam mit dem dreidimensionalen Kombinationsdatensatz.

Fig. 6 zeigt dann die für alle drei Varianten gemeinsame weitere Auswertung, die mit dem Schritt S3 - Zusammenstellung und Bereitstellung der Eingangsdaten - beginnt. Dabei sei an dieser Stelle zunächst allerdings noch angemerkt, dass, obwohl sich das Risser-Zeichen ja auf das Darmbein, insbesondere den Darmbeinkamm, als relevantes anatomisches Merkmal zur Definition bezieht, der interessierende Bereich größer gewählt ist und auch weitere anatomische Merkmale umfasst. Denn diese weiteren anatomischen Merkmale können Zusatzinformationen bereitstellen, die ebenso nützlich in der Bestimmung der Kategorie des Risser-Zeichens, also der Knocheninformation, sind.

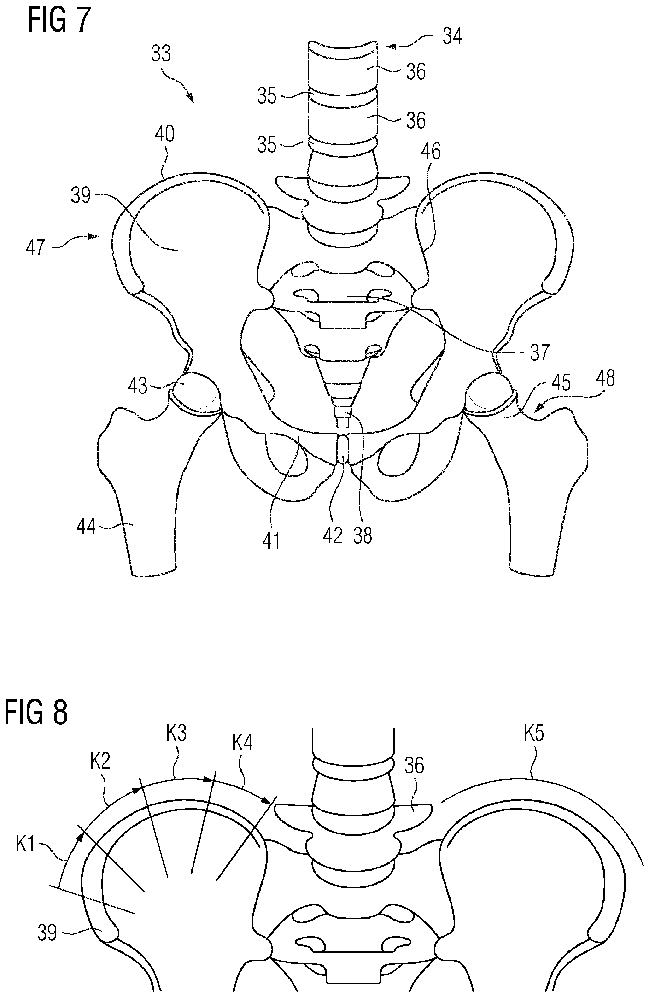

Dies sei anhand von Fig. 7 näher erläutert, die schematisch die Knochen eines Beckenbereichs als interessierender Bereich 33 zeigt. Erkennbar ist zunächst das untere Ende der Wirbelsäule 34 mit Bandscheiben 35, Lendenwirbeln 36, Kreuzbein 37 und Steißbein 38. Wesentlich für das Risser-Zeichen ist das Darmbein 39 (Ilium), da das Risser-Zeichen die Ausbildung des Darmbeinkamms 40 (oft auch einfach Beckenkamm) als Knochen betrifft, wie Fig. 8 näher erläutert. Dabei sind die verschiedenen Kategorien zur besseren Unterscheidung von sonstigen Bezugszeichen in Fig. 8 mit einem vorgestellten "K" gekennzeichnet. Hierbei werden üblicherweise folgende Kategorien von 0 bis 5 (hier K0 bis K5) verwendet:

Kategorie K0: Keine Ossifikation der Darmbeinapophyse,
Kategorie K1: Weniger als 25 % Ossifikation,
Kategorie K2: 25 - 50 % Ossifikation,
Kategorie K3: 50 - 75 % Ossifikation,
Kategorie K4: 75 - 100 % Ossifikation,
Kategorie K5: Vollständige Verknöcherung und Fusion der Apophyse.

Zurückkehrend zu Fig. 7 sind noch weitere anatomische Merkmale, hier konkret Knochen, gezeigt, hier konkret das Schambein 41, die Schambeinfuge 42 und die Hüftköpfe 43, welche das Gelenk zu dem Oberschenkelknochen 44 (Femur) bilden. Dieser weist proximale Epiphysen 45 auf. Ferner in Fig. 7 gekennzeichnet ist das Kreuz-Darmbein-Gelenk 46.

Ersichtlich umfasst der interessierende Bereich 33 mehr als nur den Darmbeinkamm 40 als definierendes anatomisches Merkmal 47, sondern zusätzliche, benachbarte anatomische Merkmale 48, wie beispielsweise die proximalen Femurepiphysen 45, welche von Radiologen auch zur Beurteilung des Risser-Zeichens berücksichtigt werden. Somit kann auch der trainierte Auswertungsalgorithmus, der gemäß Fig. 6 im Schritt S8 auf die Eingangsdaten angewendet wird, derartige Merkmale und gegebenenfalls weitere Zusammenhänge im Umfeld des definierenden anatomischen Merkmals 47 berücksichtigen.

Im vorliegenden Fall umfasst die trainierte Auswertungsfunktion, welche im Schritt S6 auf die Eingangsdaten angewendet wird, ein Convolutional Neural Network 22, welches bevorzugt als ein DenseNet ausgebildet sein kann. Die trainierte Auswertungsfunktion ermittelt aus den im Schritt S3 bereitgestellten Eingangsdaten eine Knocheninformation umfassend vorliegend die Kategorie des Risser-Zeichens als Skelettreifemaß. Hierfür sind DenseNets besonders geeignet, da es sich um eine Klassifizierung in eine der bezüglich Fig. 8 dargestellten Kategorien 0 bis 5 handelt. Dabei kann die trainierte Auswertungsfunktion auch ausgebildet sein, Zwischenwerte zu ermitteln, beispielsweise Kategorie 1.8 oder dergleichen. Die Auswertungsfunktion wurde zuvor mittels spektralen Röntgendaten, denen durch Experten Risser-Zeichen-Kategorien zugeordnet wurden, trainiert.

Nachdem das Risser-Zeichen aufgrund von spektralen Röntgendaten klassifiziert wird, geht neben der Knochenmorphologie auch die Knochendichte, insbesondere in Form des Knochenmineralgehalts (BMC), ein. Dies ist dadurch motiviert, dass die Skelettreife durch den Knochenwachstumsprozess beeinflusst wird, welcher wiederum auf dem lokalen Knochenmineralgehalt basiert. Daher wird in den hier dargestellten bevorzugten Ausführungsbeispielen auch ein speziell auf die Hervorhebung des Knochenmineralgehalts abgestimmtes Kombinationsbild in den Eingangsdaten verwendet, welches, wie aus der Röntgen-Absorptiometrie (DXA) bekannt, durch Kombination eines Hochenergie-Röntgenbilddatensatzes mit einem Niedrigenergie-Röntgenbilddatensatz bei entsprechender Gewichtung ermittelt werden kann, um insbesondere Signalanteile von Hydroxylapatit hervorzuheben. Dies geschieht, wie bereits dargelegt, im Schritt S3.

Selbstverständlich gelten diese Ausführungen auch für andere Knochendichteinformationen, insbesondere Skelettreifemaße, entsprechend, sodass auch dort durch Hinzunahme von Informationen zur Energieabhängigkeit ein Nutzen zur genauen, robusten und zuverlässigen Bestimmung der Knocheninformation entsteht.

In einem Schritt S9 wird die Knocheninformation, hier die Klassifizierung, also Kategorie, des Risser-Zeichens, dann ausgegeben, beispielsweise an einem Monitor gemeinsam mit dem Gesamtbild bzw. Röntgendaten. Auch ein Speichern in einem Speichermittel zur späteren Verwendung, eine Übertragung, insbesondere an einen Befundungsarbeitsplatz oder ein Archivierungssystem, und dergleichen sind selbstverständlich denkbar.

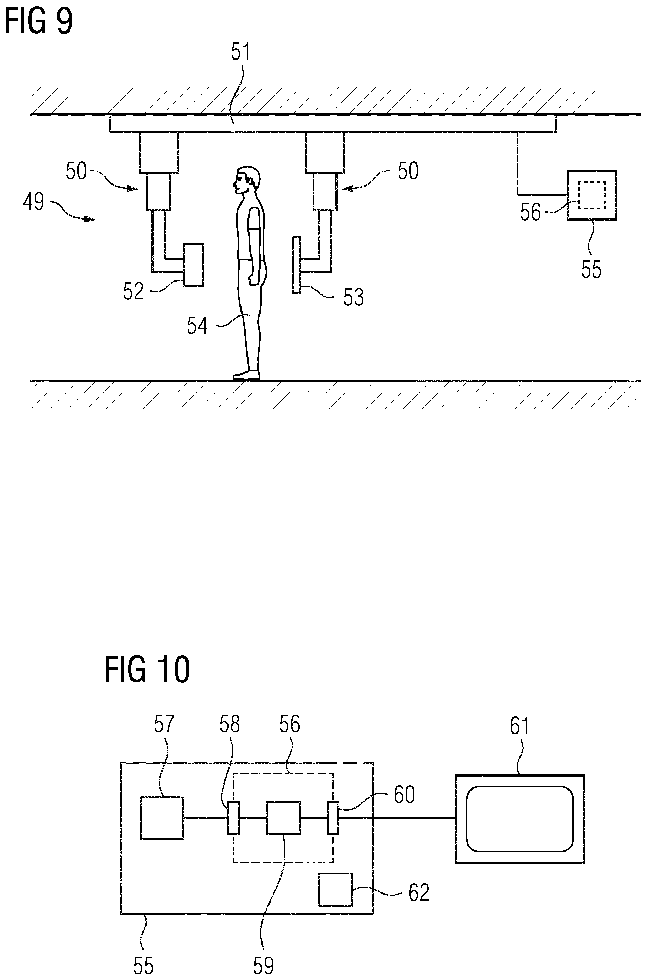

Fig. 9 zeigt schematisch ein Ausführungsbeispiel einer erfindungsgemäßen Röntgeneinrichtung 49, die vorliegend als deckenmontiertes, robotisches Bildgebungssystem ausgebildet ist. Dabei können beispielsweise robotische, hier teleskopierend ausgebildete Arme 50 in einem Schienensystem 51 geführt sein und als Teile der Aufnahmeanordnung einen Röntgenstrahler 52 und einen spektral selektiven Röntgendetektor 53, hier einen Zwei-Schicht-Detektor, tragen. Ein Patient 54 kann, zur Beurteilung der Skoliose stehend, zwischen Röntgenstrahler 52 und dem Röntgendetektor 53 angeordnet werden, wobei aufgrund der Bewegbarkeit sowohl des Röntgenstrahlers 52 als auch des Röntgendetektors 53 in verschiedenen, insbesondere allen, Freiheitsgraden sowohl ein Schlitz-Scannen unter Nutzung eines entsprechenden, hier nicht näher gezeigten Kollimators, als auch eine Tomosynthese möglich sind.

Der Betrieb der Röntgeneinrichtung 49 wird durch eine Steuereinrichtung 55 gesteuert, welche vorliegend auch eine erfindungsgemäße Ermittlungseinrichtung 56 umfasst. Fig. 10 zeigt die funktionale Struktur der Steuereinrichtung 55 in diesem Ausführungsbeispiel genauer. Die Steuereinrichtung 55 umfasst zunächst eine Aufnahmeeinheit 57, die den Aufnahmebetrieb der Röntgeneinrichtung 49 steuert. Das bedeutet, dass sie insbesondere auch die Aufnahmevorgänge, wie sie in den Figuren 3, 4 und 5 dargelegt wurden, mithin die Schritte S1 bis S7, steuern kann.

Wie bezüglich des Schrittes S3 beschrieben, werden die zusammengestellten Eingangsdaten dann über eine Schnittstelle 58, hier eine interne Schnittstelle, der Ermittlungseinrichtung 56 bereitgestellt und von einer Auswertungseinheit 59 genutzt, um, wie gemäß Schritt S8 beschrieben, den trainierten Auswertungsalgorithmus anzuwenden. Die ermittelte Knocheninformation als Ausgangsdaten wird über eine zweite Schnittstelle 60 der Ermittlungseinrichtung 56 bereitgestellt. Sie kann beispielsweise über einen Monitor 61 der Röntgeneinrichtung 49 ausgegeben werden oder aber in einem Speichermittel 62 der Steuereinrichtung 55 gespeichert werden, welches auch zumindest teilweise der Ermittlungseinrichtung 56 zugehörig sein kann.

In dem Speichermittel kann die genutzte Variante zur Aufnahme der Röntgendaten, beispielsweise eine der Varianten der Figuren 3 - 5, als ein Untersuchungsprogramm, im vorliegenden Beispielfall für Skoliose, gespeichert sein, wobei das Untersuchungsprogramm in seinem Ablauf sowohl das Gesamtbild des Aufnahmebereichs, wie auch die Knocheninformation liefert. Es sei darauf hingewiesen, dass selbstverständlich auch weitere automatische Auswertungsalgorithmen seitens der Steuereinrichtung 55 im Rahmen des Untersuchungsprogramms eingesetzt werden können, beispielsweise zur Beurteilung des aktuellen Schweregrades der Skoliose, insbesondere zur Berechnung des Cobb-Winkels, aber auch zur Ermittlung weiterer Informationen zur Beurteilung des Risikos eines Fortschreitens der Skoliose, wobei grundsätzlich auch eine Risikoabschätzung unter Nutzung auch der Knocheninformation denkbar ist.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

1.  Computerimplementiertes Verfahren zur Ermittlung einer die Skelettreife eines Patienten (54) beschreibenden Knocheninformation, umfassend die Schritte:

    - Bereitstellung von Eingangsdaten, umfassend spektral aufgelöste Röntgendaten eines zur Beurteilung der Skelettreife heranzuziehenden interessierenden Bereichs (33) des Patienten (54) mit wenigstens zwei auf unterschiedliche Röntgenspektren bezogenen Röntgenbilddatensätzen,
    - Anwenden einer trainierten Auswertungsfunktion auf die Eingangsdaten zum Erhalt der Knocheninformation als Ausgangsdaten, und
    - Ausgabe der Knocheninformation,

    wobei die Röntgendaten wenigstens teilweise als Teil oder im Rahmen eines einen größeren Aufnahmebereich als den interessierenden Bereich (33) betreffenden Untersuchungsvorgangs aufgenommen werden, wenigstens ein zweidimensional aufgenommenes Röntgenbild des Untersuchungsvorgangs und/oder eine empfangene Nutzerinformation zur Lokalisierung des interessierenden Bereichs (33) ausgewertet werden, wobei das Auswertungsergebnis zur nachfolgenden oder unterbrechenden Aufnahme dreidimensionaler, nur auf den interessierenden Bereich (33) bezogener dreidimensionaler Röntgendaten und/oder zur Auswahl von als Eingangsdaten zu verwendenden zweidimensionalen Röntgendaten der Röntgenbilder verwendet werden.

2.  Verfahren nach Anspruch 1, **dadurch gekennzeichnet, dass** als Eingangsdaten auch ein aus den Röntgenbilddatensätzen ermittelter, Knochenmineralien hervorhebender Kombinationsbilddatensatz verwendet wird.

3.  Verfahren nach Anspruch 1 oder 2, **dadurch gekennzeichnet, dass** die Röntgendaten mittels eines spektral selektiven Röntgendetektors (53) aufgenommen werden.

4.  Verfahren nach einem der vorangehenden Ansprüche, **dadurch gekennzeichnet, dass** jeweils wenigstens zwei zweidimensionale und/oder jeweils wenigstens zwei dreidimensionale Röntgenbilddatensätze für die unterschiedlichen Spektren bereitgestellt werden.

**5.** Verfahren nach Anspruch 4, **dadurch gekennzeichnet, dass** die zweidimensionalen Röntgenbilddatensätze mittels Schlitzscannen aufgenommen werden und/oder die dreidimensionalen Röntgenbilddatensätze durch Tomosynthese aufgenommen werden.

**6.** Verfahren nach einem der vorangehenden Ansprüche, **dadurch gekennzeichnet, dass** aus den dreidimensionalen Röntgendaten ein zweidimensionales Synthetikbild des interessierenden Bereichs (33) ermittelt wird, welches mit dem wenigstens einen Röntgenbild fusioniert wird.

**7.** Verfahren nach einem der vorangehenden Ansprüche, **dadurch gekennzeichnet, dass** bei einer sich auf ein bestimmtes anatomisches Merkmal (47) beziehenden Knocheninformation der interessierende Bereich (33) umfassend wenigstens ein Zusatzinformation bereitstellendes, zusätzliches, benachbartes anatomisches Merkmal (48) gewählt wird.

**8.** Verfahren nach einem der vorangehenden Ansprüche, **dadurch gekennzeichnet, dass** die trainierte Auswertungsfunktion ein Convolutional Neural Network, insbesondere ein DenseNet, umfasst.

**9.** Verfahren nach einem der vorangehenden Ansprüche, **dadurch gekennzeichnet, dass** als Knocheninformation ein Skelettreifemaß ermittelt wird.

**10.** Röntgeneinrichtung (49), aufweisend einen Röntgenstrahler (52) und einen insbesondere spektral selektiven Röntgendetektor (53) sowie eine zur Durchführung eines Verfahrens nach einem der vorangehenden Ansprüche ausgebildete Steuereinrichtung (55).

**11.** Ermittlungseinrichtung (46) zur Ermittlung einer die Skelettreife eines Patienten (54) beschreibenden Knocheninformation, aufweisend:

- eine erste Schnittstelle (58) zur Bereitstellung von Eingangsdaten, umfassend spektral aufgelöste Röntgendaten eines zur Beurteilung der Skelettreife heranzuziehenden interessierenden Bereichs (33) des Patienten (54) mit wenigstens zwei auf unterschiedliche Röntgenspektren bezogenen Röntgenbilddatensätzen,
- eine Auswertungseinheit (59) zum Anwenden einer trainierten Auswertungsfunktion auf die Eingangsdaten zum Erhalt der Knocheninformation als Ausgangsdaten, und
- eine zweite Schnittstelle (60) zur Ausgabe der Knocheninformation,

wobei die Röntgendaten wenigstens teilweise als Teil oder im Rahmen eines einen größeren Aufnahmebereich als den interessierenden Bereich (33) betreffenden Untersuchungsvorgangs aufgenommen werden,
wenigstens ein zweidimensional aufgenommenes Röntgenbild des Untersuchungsvorgangs und/oder eine empfangene Nutzerinformation zur Lokalisierung des interessierenden Bereichs (33) ausgewertet werden, wobei das Auswertungsergebnis zur nachfolgenden oder unterbrechenden Aufnahme dreidimensionaler, nur auf den interessierenden Bereich (33) bezogener dreidimensionaler Röntgendaten und/oder zur Auswahl von als Eingangsdaten zu verwendenden zweidimensionalen Röntgendaten der Röntgenbilder verwendet werden.

**12.** Computerprogramm, welches die Schritte eines Verfahrens nach einem der Ansprüche 1 bis 9 durchführt, wenn es auf einer Recheneinrichtung ausgeführt wird.

**13.** Elektronisch lesbarer Datenträger, auf dem ein Computerprogramm nach Anspruch 12 gespeichert ist.

**Claims**

**1.** Computer-implemented method for determining an item of bone information describing the skeletal maturity of a patient (54), comprising the steps:

- providing input data, comprising spectrally resolved X-ray data of a region of interest (33) of the patient (54) to be used for assessing the skeletal maturity, with at least two X-ray image datasets relating to X-ray spectra,
- applying a trained evaluation function to the input data to obtain bone information as output data, and
- outputting the bone information,

wherein

the X-ray data is acquired at least to some extent as part of or in the context of an examination procedure relating to an acquisition region that is larger than the region of interest (33),

at least an X-ray image of the examination procedure which is acquired in two dimensions and/or an item of user information received for localising the region of interest (33) is evaluated, wherein the evaluation result is used for subsequent or intermittent acquisition of three-dimensional X-ray data relating only to the region of interest (33) and/or for selecting two-dimensional X-ray data of the X-ray images to be used as input data.

2. Method according to claim 1, **characterised in that** a combination image dataset determined from the X-ray image datasets which highlights bone minerals is also used as input data.

3. Method according to claim 1 or 2, **characterised in that** the X-ray data is acquired by means of a spectrally selective X-ray detector (53).

4. Method according to one of the preceding claims, **characterised in that** at least two two-dimensional and/or at least two three-dimensional X-ray image datasets are provided in each case for the different spectra.

5. Method according to claim 4, **characterised in that** the two-dimensional X-ray image datasets are acquired by means of slit scanning and/or the three-dimensional X-ray image datasets are acquired by way of tomosynthesis.

6. Method according to one of the preceding claims, **characterised in that** a two-dimensional synthetic image of the region of interest (33) is determined from the three-dimensional X-ray data, which image is fused with the at least one X-ray image.

7. Method according to one of the preceding claims, **characterised in that** the region of interest (33), comprising an additional adjacent anatomical feature (48) that provides at least one item of additional information, is selected in the case of an item of bone information relating to a specific anatomical feature (47).

8. Method according to one of the preceding claims, **characterised in that** the trained evaluation function comprises a convolutional neural network, in particular a DenseNet.

9. Method according to one of the preceding claims, **characterised in that** a degree of skeletal maturity is determined as bone information.

10. X-ray facility (49), having an X-ray emitter (52) and an X-ray detector (53) which is in particular spectrally selective, and a control facility (55) designed to carry out a method according to one of the preceding claims.

11. Determination facility (46) for determining an item of bone information describing the skeletal maturity of a patient (54), having:

   - a first interface (58) for providing input data, comprising spectrally resolved X-ray data of a region of interest (33) of the patient (54) to be used for assessing the skeletal maturity with at least two X-ray image datasets relating to different X-ray spectra,
   - an evaluation unit (59) for applying a trained evaluation function to the input data to obtain the bone information as output data, and
   - a second interface (60) for outputting the bone information, wherein
   the X-ray data is acquired at least partially as part of or in the context of an examination procedure relating to an acquisition region that is larger than the region of interest (33),
   at least an X-ray image of the examination procedure that is acquired in two dimensions and/or an item of user information received for localising the region of interest (33) is evaluated, wherein the evaluation result is used for subsequent or intermittent acquisition of three-dimensional X-ray data relating only to the region of interest (33) and/or for selecting two-dimensional X-ray data of the X-ray images to be used as input data.

12. Computer program, which carries out the steps of a method according to one of claims 1 to 9 when it is executed on a computer facility.

13. Electronically readable data carrier, on which a computer program according to claim 12 is stored.

**Revendications**

1. Procédé mis en œuvre par ordinateur de détermination d'une information osseuse décrivant la maturité du squelette d'un patient (54), comprenant les stades :

   - se procurer des données d'entrée comprenant des données de rayons X à résolution spectrale d'une zone (33) du patient (54) intéressante, dont on tire parti pour juger de la maturité du squelette, comprenant au moins deux ensembles de données d'image de rayons X rapportés à des spectres de rayons X différents,
   - appliquer une fonction d'évaluation entraînée aux données d'entrée pour obtenir l'information osseuse comme données de sortie, et
   - sortir l'information osseuse,

   dans lequel

   les données de rayons X sont enregistrées au moins en partie comme partie ou dans le cadre d'une zone d'enregistrement plus grande que l'opération d'examen concernant la zone (33) intéressante, on évalue au moins une image de rayons X enregistrée en deux dimensions de l'opération d'examen et/ou une information d'utilisateur reçue pour la localisation de la zone (33) intéressante, dans lequel on n'utilise le résultat de l'évaluation pour l'enregistrement venant ensuite ou interrompu en trois dimensions que sur les données de rayons X en trois dimensions se rapportant à la zone (33) intéressante et/ou que pour la détection de données de rayons X, bidimensionnelles à utiliser comme données d'entrée, des images de rayons X.

2. Procédé suivant la revendication 1, **caractérisé en ce que** l'on utilise comme données d'entrée également un ensemble de données d'image de combinaisons déterminé à partir des ensembles de données d'image de rayons X et mettant en exergue des minéraux de l'os.

3. Procédé suivant la revendication 1 ou 2, **caractérisé en ce que** l'on enregistre les données de rayons X au moyen d'un détecteur (53) de rayons X sélectif spectralement.

4. Procédé suivant l'une des revendications précédentes, **caractérisé en ce que** l'on se procure pour les spectres différents respectivement au moins deux ensembles de données d'image de rayons X en deux dimensions et/ou respectivement en au moins trois dimensions.

5. Procédé suivant la revendication 4, **caractérisé en ce que** l'on enregistre, au moyen d'un balayage par fente les ensembles de données d'image de rayons X en deux dimensions et/ou on enregistre par tomosynthèse les ensembles de données d'image de rayons X en trois dimensions.

6. Procédé suivant l'une des revendications précédentes, **caractérisé en ce qu'**à partir des données de rayons X en trois dimensions, on détermine une image synthétique en deux dimensions de la zone (33) intéressante, que l'on fusionne avec la au moins une image de rayons X.

7. Procédé suivant l'une des revendications précédentes, **caractérisé en ce que** pour une information osseuse, se rapportant à une caractéristique (47) anatomique déterminée, de la région (33) intéressante, on choisit une caractéristique (48) anatomique voisine, supplémentaire donnant au moins une information supplémentaire.

8. Procédé suivant l'une des revendications précédentes, **caractérisé en ce que** la fonction d'évaluation entraînée comprend un réseau neuronal de convolution, en particulier un DenseNet.

9. Procédé suivant l'une des revendications précédentes, **caractérisé en ce que** l'on détermine, comme information osseuse, une mesure de maturité du squelette.

10. Dispositif (49) de rayons X, comportant un émetteur (52) de rayons X et un détecteur (53) de rayons X, en particulier sélectif spectralement ainsi qu'un dispositif (55) de commande constitué pour effectuer un procédé suivant l'une des revendications précédentes.

11. Dispositif (46) de détermination d'une information osseuse décrivant la maturité du squelette d'un patient (54), comportant :

- une première interface (58) pour donner des données d'entrée, comprenant des données de rayons X à résolution spectrale d'une zone (33) du patient (54) intéressante, dont on tire parti pour juger de la maturité du squelette, comprenant au moins deux ensembles de données d'image de rayons X rapportés à des spectres de rayons X différents,
- une unité (59) d'évaluation pour appliquer une fonction d'évaluation entraînée aux données d'entrée, afin d'obtenir l'information osseuse comme données de sortie, et
- une deuxième interface (60) de sortie de l'information osseuse,

dans lequel

les données de rayons X sont enregistrées au moins en partie comme partie ou dans le cadre d'une zone d'enregistrement plus grande que l'opération d'examen concernant la zone (33) intéressante, au moins une image de rayons X enregistrée en deux dimensions de l'opération d'examen et/ou une information d'utilisateur reçue pour la localisation de la zone (33) intéressante sont évaluées, dans lequel le résultat de l'évaluation pour l'enregistrement venant ensuite ou interrompu en trois dimensions n'est utilisé que sur les données de rayons X en trois dimensions se rapportant à la zone (33) intéressante et/ou que pour la détection de données de rayons X, bidimensionnelle à utiliser comme données d'entrée, des images de rayons X.

12. Programme d'ordinateur, qui effectue les stades d'un procédé suivant l'une des revendications 1 à 9, lorsqu'il est exécuté sur un dispositif informatique.

13. Support de données déchiffrable électroniquement, sur lequel un programme d'ordinateur suivant la revendication 12 est mis en mémoire.

FIG 1

# FIG 2

FIG 3

```
┌──────────────┐
│              │ ──⌒── S1
└──────────────┘
       │
       ▼
┌──────────────┐
│              │ ──⌒── S2
└──────────────┘
       │
       ▼
┌──────────────┐
│              │ ──⌒── S3
└──────────────┘
```

FIG 4

```
┌──────────────┐
│              │ ──⌒── S1
└──────────────┘
       │
       ▼
┌──────────────┐
│              │ ──⌒── S2
└──────────────┘
       │
       ▼
┌──────────────┐
│              │ ──⌒── S4
└──────────────┘
       │
       ▼
┌──────────────┐
│              │ ──⌒── S3
└──────────────┘
```

## FIG 5

S1

S5

S4

S6

S1

S7

S3

## FIG 6

S3

S8

S9

## FIG 7

## FIG 8

FIG 9

FIG 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **SABRINA DONZELLI et al.** Predicting scoliosis progression: a challenge for researchers and clinicians. *EClinicalMedicine*, 2020, vol. 18, 100244
- **MAXIMILIAN LENZ et al.** Scoliosis and Prognosis - a systematic review regarding patient-specific and radiological predictive factors for curve progression. *European Spine Journal*
- **JAE HYUK YANG et al.** Evaluation of accuracy of plain radiography in determining the Risser stage and identification of common sources of errors. *Journal of Orthopaedic Surgery and Research*, 2014, vol. 9, 101

- **HOUDA KADDIOUI et al.** Convolutional Neural Networks for Automatic Risser Stage Assessment. *Radiology: Artificial Intelligence*, 2020, vol. 2 (3)
- **MARTIN THALER et al.** Radiographic versus ultrasound evaluation of the Risser Grade in adolescent idiopathic scoliosis: a prospective study of 46 patients. *European Spine Journal*, vol. 17 (9), 1251-1255
- *Deep Transfer Learning*, 2021
- Dual Energy X-ray Absorptiometry for Bone Mineral Density and Body Composition Assessment. *IAEA*, 2010, https://www-pub.iaea.org/MTCD/Publications/PDF/Pub1479_web.pdf